# EUROPEAN PATENT SPECIFICATION

(11) **EP 1 575 949 B1**
(45) Date of publication and mention of the grant of the patent: **02.04.2008**
(21) Application number: 03780337.6
(22) Date of filing: 04.12.2003
(51) Int. Cl.: C07D 471/04, A01N 43/90, C07D 239/42

(54) **FUNGICIDES**
FUNGIZIDE
FONGICIDES

(30) Priority: 23.12.2002 GB 0230019
(43) Date of publication of application: 21.09.2005
(73) Proprietor: Syngenta Limited, Guildford Surrey GU2 7YH (GB); Syngenta Participations AG, 4058 Basel (CH)
(72) Inventor: CROWLEY, Patrick Jelf, Syngenta Limited, Bracknell, Berks RG42 6EY (GB); DOBLER, Markus, Novartis Inst. for BioMedical, Cambridgr, MA 02139 (US); MUELLER, Urs, 4058 Basel (CH); WILLIAMS, John, Syngenta Limited, Bracknell, Berks RG42 6EY (GB)
(74) Representative: Arunasalam, Velautha-Cumaran
(86) International application number: PCT/GB2003/005273
(87) International publication number: WO 2004/056826

(56) References cited:
- EP-A- 0 537 463
- DORNOW A ET AL: "UBER ORTHO-KONDENSATIONEN HETEROCYCLISCHER O-AMINO-CARBONSAURE- DERIVATE" CHEMISCHE BERICHTE, VERLAG CHEMIE GMBH. WEINHEIM, DE, vol. 91, no. 18, 1958, pages 1834-1840, XP000561883 ISSN: 0009-2940
- DATABASE CHEMCATS [Online] Chemical Abstract Service, Columbus, Ohio, US; retrieved from STN Database accession no. 2002:2623896 XP002271641 & "Interchim Intermediates" 9 July 2002 (2002-07-09) , INTERCHIM , MONTLUCON, CEDEX 03103, FRANCE

## Description

This invention relates to novel derivatives of pyridopyrimidines, to processes for preparing them, to certain intermediate chemicals used in their manufacture, to compositions containing them and to methods of using them to combat fungi, especially fungal infections of plants.

Derivatives of the nitrogen-containing 5,6 ring system *s*-1,2,4-triazolo[1,5-a]pyrimidine are known from the patent literature as being useful for controlling phytopathogenic fungi. Examples of recent patent publications include EP-A-1249452, WO 02/051845, WO 02/083676, WO 02/083677, WO 02/088125, WO 02/088126, WO 02/088127. Certain derivatives of pyridopyrimidines are described, for example, in US 5597776 and WO 01/17972 as herbicide antagonists. Others are known for pharmaceutical applications (see, for example, J. Med. Chem. (1983), 26(3), 403).

The present invention is concerned with the provision of novel pyridopyrimidines for combating phytopathogenic diseases on plants and harvested food crops.
Thus, according to the present invention, there is provided a compound of the general formula (1): 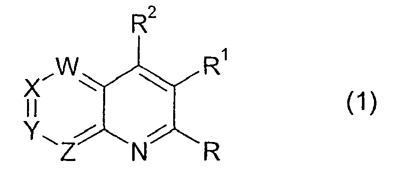 wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
R and R² are independently H, halo, C₁₋₈ alkyl, C₁₋₈ alkoxy, C₁₋₈ alkylthio, C₂₋₈ alkenyl, C₂₋₈ alkynyl, cyano or NR³R⁴, provided that at least one of R and R² is NR³R⁴;
R¹ is halo, C₁₋₈ alkyl, C₂₋₈ alkenyl, C₂₋₈ alkynyl, C₃₋₈ cycloalkyl, C₃₋₈ cycloalk-yl(C₁₋₆)alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl(C₁₋₄)alkoxy, aryl(C₁₋₄)alkylthio, heteroaryl(C₁₋₄)alkylthio, morpholino, piperidino or pyrrolidino;
R³ and R⁴ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are H or NR⁵R⁶, or
R³ and R⁴ together form a C₃₋₇ alkylene or C₃₋₇ alkenylene chain optionally substituted with one or more C₁₋₄ alkyl or C₁₋₄ alkoxy groups, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring; and
R⁵ and R⁶ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl or heteroaryl(C₁₋₈)alkyl;
any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and
any of the foregoing aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy(C₁₋₆₎alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R''', -OSO₂R''', -COR''', -CR'''=NR"" or -N=CR'''R"", in which R"' and R"" are independently hydrogen, C₁₋₄ alkyl, halo(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy;
provided that Y is not CCH₃ when W is CH, X and Z are N, R is NHCH₃, R¹ is 2,6-dichlorophenyl and R² is H.

The invention includes a compound of the general formula (1) as defined immediately above except that: C₁₋₈ alkoxy and C₁₋₈ alkylthio are excluded as values of R and R²; C₇ alkylene and C₃₋₇ alkenylene are excluded as chains formed by R³ and R⁴; the C₃₋₆ chain that R³ and R⁴ may form may only be optionally substituted with one or more methyl groups; thiomorpholine, thiomorpholine S-oxide, thiomorpholine S-dioxide and piperazine are excluded as rings that R³ and R⁴ may form; tri(C₁₋₄)alkylsilyl is excluded as a substituent of any alkyl, alkenyl, alkynyl or cycloalkyl group or moiety and any morpholine, piperidine or pyrrolidine ring is unsubstituted.

The invention also includes a compound of the general formula (1) as defined above where R² is other than H and also a compound of the general formula (1) as defined above where R⁸ is H.

The compounds of the invention may contain one or more asymmetric carbon atoms and may exist as enantiomers (or as pairs of diastereoisomers) or as mixtures of such. They may also exist as diastereoisomers by virtue of restricted rotation about a bond. However, mixtures of enantiomers or diastereoisomers may be separated into individual isomers or isomer pairs, and this invention embraces such isomers and mixtures thereof in all proportions. It is to be expected that for any given compound, one isomer may be more fungicidally active than another.

Except where otherwise stated, alkyl groups and alkyl moieties of alkoxy, alkylthio, etc., contain from 1 to 8, suitably from 1 to 6 and typically from 1 to 4, carbon atoms in the form of straight or branched chains. Examples are methyl, ethyl, *n*- and *iso*-propyl, *n*-, *sec*-, *iso*- and *tert*-butyl, *n*-pentyl and *n*-hexyl. Cycloalkyl groups contain from 3 to 8, typically from 3 to 6, carbon atoms and include bicycloalkyl groups such as the bicyclo[2.2.1]heptyl group. Haloalkyl groups or moieties are typically trichloromethyl or trifluoromethyl or contain a trichloromethyl or trifluoromethyl terminal group.

Except where otherwise stated, alkenyl and alkynyl moieties also contain from 2 to 8, suitably from 2 to 6 and typically from 2 to 4, carbon atoms in the form of straight or branched chains. Examples are allyl, 2-methylallyl and propargyl. Optional substituents include halo, typically fluoro. An example of halo-substituted alkenyl is 3,4,4-trifluoro-n-butenyl.

Halo includes fluoro, chloro, bromo and iodo. Most commonly it is fluoro, chloro or bromo and usually fluoro or chloro.

Aryl is usually phenyl but also includes naphthyl, anthryl and phenanthryl.

Heteroaryl is typically a 5- or 6-membered aromatic ring containing one or more O, N or S heteroatoms, which may be fused to one or more other aromatic or heteroaromatic rings, such as a benzene ring. Examples are thienyl, furyl, pyrrolyl, isoxazolyl, oxazolyl, oxadiazolyl, pyrazolyl, imidazolyl, triazolyl, isothiazolyl, tetrazolyl, thiadiazolyl, pyridyl, pyrimidinyl, pyrazinyl, pyridazinyl, triazinyl, benzofuryl, benzothienyl, dibenzofuryl, benzothiazolyl, benzoxazolyl, benzimidazolyl, indolyl, quinolinyl and quinoxalinyl groups and, where appropriate, N-oxides thereof.

The 6,6-ring systems embraced by the general formula (1) are pyrido[2,3-d]pyrimidines (where W and Y are both CR⁸ and X and Z are both N) and pyrido[3,2-d]pyrimidines (where X and Z are both CR⁸ and W and Y are both N).

R⁸, which may be the same or different for the two CR⁸ values of W, X, Y and Z, is H, halo (for example bromo), C₁₋₄ alkyl (for example methyl), C₁₋₄ alkoxy (for example methoxy) or halo(C₁₋₄)alkyl (for example trifluoromethyl). Usually R⁸ will be H.

One of R and R², preferably R², is NR³R⁴. The other is typically halo, especially chloro or fluoro. In the case of pyrido[3,2-d]pyrimidine ring systems, the more active compounds are those where R² is NR³R⁴. R³ is typically C₁₋₈ alkyl (for example ethyl, n-propyl, *n*-butyl, *sec*-butyl (the S- or R-isomer or the racemate) and *tert*-butyl), halo(C₁₋₈)alkyl (for example 2,2,2-trifluoroethyl, 2,2,2-trifluoro-1-methylethyl (the S- or R-isomer or the racemate), 3,3,3-trifluoropropyl and 4,4,4-trifluorobutyl), hydroxy(C₁₋₈)alkyl (for example hydroxyethyl), C₁₋₄ alkoxy(C₁₋₈)alkyl (for example methoxymethyl and methoxy-*iso*-butyl), C₁₋₄ alkoxyhalo(C₁₋₈)alkyl (for example 2-methoxy-2-trifluoromethylethyl), tri(C₁₋₄)-alkylsilyl(C₁₋₆)alkyl (for example trimethylsilylmethyl), C₁₋₄ alkylcarbonyl(C₁₋₈)alkyl (for example 1-acetylethyl and 1-*tert*-butylcarbonylethyl), C₁₋₄ alkylcarbonylhalo(C₁₋₈)alkyl (for example 1-acetyl-2,2,2-trifluoroethyl), phenyl(₁₋₄)alkyl (for example benzyl), C₂₋₈ alkenyl (for example allyl and methylallyl), halo(C₂₋₈)alkenyl (for example 3-methyl-4,4-difluorobut-3-enyl), C₂₋₈ alkynyl (for example propargyl), C₃₋₈ cycloalkyl (for example cyclopropyl, cyclobutyl, cyclopentyl and cyclohexyl) optionally substituted with chloro, fluoro or methyl, C₃₋₈ cycloalkyl(C₁₋₄)alkyl (for example cyclopropylmethyl, cyclobutylmethyl, cyclopentylmethyl and cyclohexylmethyl), phenylamino, piperidino or morpholino, the phenyl ring of phenylalkyl or phenylamino being optionally substituted with one, two or three substituents selected from halo (typically fluoro, chloro or bromo), C₁₋₄ alkyl (typically methyl), halo(C₁₋₄)alkyl (typically trifluoromethyl), C₁₋₄ alkoxy (typically methoxy) and halo(C₁₋₄)alkoxy (typically trifluoromethoxy). R⁴ is typically H, C₁₋₄ alkyl (for example ethyl and *n*-propyl), halo(C₁₋₄)alkyl (for example 2,2,2-trifluoroethyl) or amino. Alternatively R³ and R⁴ together form a C₄₋₆ alkylene chain optionally substituted with methyl, for example 3-methylpentylene, or, together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring, in which the morpholine or piperazine rings are optionally substituted with methyl.

Typically R¹ is phenyl optionally substituted with from one to five halogen atoms, particularly fluorine and chlorine atoms and especially fluorine atoms or with from one to three substituents selected from halo (for example fluoro and chloro), C₁₋₄ alkyl (for example methyl), halo(C₁₋₄)alkyl (for example trifluoromethyl), C₁₋₄ alkoxy (for example methoxy) or halo(C₁₋₄)alkoxy (for example trifluoromethoxy). Examples are 2,6-difluorophenyl, 2-fluoro-6-chlorophenyl, 2,5,6-trifluorophenyl, 2,4,6-trifluorophenyl, 2,6-difluoro-4-methoxyphenyl, pentafluorophenyl, 2-fluorophenyl, 2,3,5,6-tetrafluorophenyl, 2-chloro-4,6-difluorophenyl, 2-chlorophenyl, 2,6-dichlorophenyl, 2,4-dichlorophenyl, 2,4,6-trichlorophenyl, 2,3,6-trichlorophenyl, pentachlorophenyl, 2-fluoro-4,6-dichlorophenyl, 4-fluoro-2,6-dichlorophenyl, 2-bromophenyl, 2-fluoro-6-bromophenyl, 2-bromo-4,6-difluorophenyl, 2-fluoro-6-methylphenyl, 2-chloro-6-methylphenyl, 2-methoxyphenyl, 2,6-dimethoxyphenyl, 2-fluoro-6-methoxyphenyl, 2-trifluoromethylphenyl, 2-fluoro-6-trifluoromethylphenyl, 2,6-di-(trifluoromethyl)phenyl, 2-chloro-6-trifluoromethylphenyl, 2,4-difluoro-6-trifluoromethylphenyl, 2,4-difluoro-6-methoxyphenyl and 2,4-difluoro-6-methylphenyl.

Also of particular interest are compounds where R¹ is pyridyl optionally substituted with from one to four halogen atoms or with from one to three substituents selected from halo (for example fluoro and chloro), C₁₋₄ alkyl (for example methyl), halo(C₁₋₄)alkyl (for example trifluoromethyl), C₁₋₄ alkoxy (for example methoxy) or halo(C₁₋₄)alkoxy (for example trifluoromethoxy). Examples are 2,4-difluoropyrid-3-yl, 3,5-difluoropyrid-4-yl, tetrafluoropyrid-4-yl, 3-fluoropyrid-2-yl, 4-fluoropyrid-3-yl, 3-fluoropyrid-4-yl, 2-fluoropyrid-3-yl, 2,4,6-trifluoropyrid-3-yl, 3,5-difluoropyrid-2-yl, 2,6-difluoropyrid-3-yl, 2,4-difluoro-6-methoxypyrid-3-yl, 2-fluoro-4-chloropyrid-3-yl, 3-fluoro-5-chloropyrid-4-yl, 2-chloro-4-fluoropyrid-3-yl, 2,4-dichloropyrid-3-yl, 3-chloropyrid-2-yl I, 4-chloropyrid-3-yl, 3-chloropyrid-4-yl, 2-chloropyrid-3-yl, 3-trifluoromethylpyrid-2-yl, 4-trifluoromethylpyrid-3-yl, 3,5-dichloropyrid-2-yl, 4,6-dichloropyrid-3-yl, 3-trifluoromethylpyrid-4-yl, 2-trifluoromethylpyrid-3-yl, 2-fluoro-4-trifluoromethylpyrid-3-yl, 3-fluoro-S-trifluoromethylpyrid-4-yl, 4-fluoro-2-trifluoromethylpyrid-3-yl, 2,6-dichloropyrid-3-yl, 3,5-dichloropyrid-4-yl, 3-chloro-6-trifluoromethylpyrid-2-yl, 3-fluoro-6-trifluoromethylpyrid-2-yl, pyrid-2-yl, pyrid-3-yl and pyrid-4-yl.

Also of particular interest are compounds where R¹ is 2- or 3-thienyl optionally substituted with from one to three halogen atoms or with from one to three substituents selected from halo (for example fluoro and chloro), C₁₋₄ alkyl (for example methyl), halo-(C₁₋₄)alkyl (for example trifluoromethyl), C₁₋₄ alkoxy (for example methoxy) or halo(C₁₋₄)-alkoxy (for example trifluoromethoxy). Examples are 3-fluorothien-2-yl, 3-chlorothien-2-yl, 2,4-difluorothien-3-yl, 2,4-dichlorothien-3-yl and 2,4,5-trichlorothien-3-yl.

Examples of other values of R¹ of especial interest are unsubstituted piperidino and morpholino, 2-methylpiperidino, 2,6-dimethylpiperidino and 2,6-dimethylmorpholino

In one aspect the invention provides a compound of the general formula (1) wherein W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
one of R and R² (preferably R²) is NR³R⁴ and the other is halo;
R¹ is halo, C₁₋₈ alkyl, C₂₋₈ alkenyl, C₂₋₈ alkynyl, C₃₋₈ cycloalkyl, C₃₋₈ cycloalkyl(C₁₋₆)alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl(C₁₋₄)alkoxy, aryl(C₁₋₄)alkylthio, heteroaryl(C₁₋₄)alkylthio, morpholino, piperidino or pyrrolidino;
R³ and R⁴ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are H or NR⁵R⁶, or
R³ and R⁴ together form a C₃₋₇ alkylene chain or C₃₋₇ alkenylene chain optionally substituted with one or more C₁₋₄ alkyl or C₁₋₄ alkoxy groups, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N-*methyl) ring; and
R⁵ and R⁶ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl or heteroaryl(C₁₋₈)alkyl;
any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and
any of the foregoing aryl, heteroaryl, aryloxy or heteroaryl groups being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy(C₁₋₆)-alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR'''R"", -NHCOR"', -NHCONR'''R"", -CONR'''R"", -SO₂R''', -OSO₂R''', -COR''', -CR'''=NR"" or -N=CR'''R"", in which R"' and R"" are independently hydrogen, C₁₋₄ alkyl, halo(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.
Of particular interest are compounds where R⁸ is H.

The invention includes a compound of the general formula (1) as defined immediately above except that: C₇ alkylene and C₃₋₇ alkenylene are excluded as chains formed by R³ and R⁴; the C₃₋₆ chain that R³ and R⁴ may form may only be optionally substituted with one or more methyl groups; thiomorpholine, thiomorpholine S-oxide, thiomorpholine S-dioxide and piperazine are excluded as rings that R³ and R⁴ may form; tri(C₁₋₄)alkylsilyl is excluded as a substituent of any alkyl, alkenyl, alkynyl or cycloalkyl group or moiety, and any morpholine, piperidine or pyrrolidine ring is unsubstituted.

In another aspect the invention provides a compound of the general formula (1) wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
one of R and R² (preferably R²) is NR³R⁴ and the other is halo;
R¹ is halo, C₁₋₈ alkyl, C₂₋₈ alkenyl, C₂₋₈ alkynyl, C₃₋₈ cycloalkyl, C₃₋₈ cycloalkyl(C₁₋₆)alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl(C₁₋₄)alkoxy, aryl(C₁₋₄)alkylthio, heteroaryl(C₁₋₄)alkylthio, morpholino, piperidino or pyrrolidino;
R³ is C₁₋₄ alkyl, halo(C₁₋₄)alkyl, C₂₋₄ alkenyl, C₃₋₆ cycloalkyl, C₃₋₆ cycloalkyl(C₁₋₄)alkyl or phenylamino in which the phenyl ring is optionally substituted with one, two or three substituents selected from halo, C₁₋₄ alkyl, halo(C₁₋₄)alkyl, C₁₋₄ alkoxy and halo(C₁₋₄)alkoxy; and
R⁴ is H, C₁₋₄ alkyl or amino, or
R³ and R⁴ together form a C₄₋₆ alkylene chain optionally substituted with C₁₋₄ alkyl or C₁₋₄ alkoxy, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring;
any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and
any of the foregoing aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR'''R'''', -NHCOR"', -NHCONR'''R"", -CONR'''R"", -SO₂R''', -OSO₂R''', -COR''', -CR'''=NR"" or -N=CR'''R'''', in which R"' and R"" are independently hydrogen, C₁₋₄ alkyl, halo(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.
Of particular interest are compounds where R⁸ is H.

The invention includes a compound of the general formula (1) as defined immediately above except that: the C₄₋₆ chain that R³ and R⁴ may form may only be optionally substituted with methyl; thiomorpholine, thiomorpholine S-oxide, thiomorpholine S-dioxide and piperazine are excluded as rings that R³ and R⁴ may form; tri(C₁₋₄)alkylsilyl is excluded as a substituent of any alkyl, alkenyl, alkynyl or cycloalkyl group or moiety, and any morpholine, piperidine or pyrrolidine ring is unsubstituted.

In yet another aspect the invention provides a compound of the general formula (1) wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
R and R² are independently H, halo, C₁₋₈ alkyl, C₁₋₈ alkoxy, C₁₋₈ alkylthio, C₂₋₈ alkenyl, C₂₋₈ alkynyl, cyano or NR³R⁴, provided that at least one of R and R² (preferably R²) is NR³R⁴; R¹ is optionally substituted phenyl;
R³ and R⁴ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are H or NR⁵R⁶, or
R³ and R⁴ together form a C₃₋₇ alkylene or C₃₋₇ alkenylene chain optionally substituted with one or more C₁₋₄ alkyl or C₁₋₄ alkoxy groups, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring; and
R⁵ and R⁶ are independently H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl or heteroaryl(C₁₋₈)alkyl;
any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and
any of the foregoing aryl or heteroaryl groups or moieties, including the phenyl group of R¹, being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy(C₁₋₆)alkyl, C₃₋₆ cycloalkyl, C₃₋₆ cycloalkyl(C₁-₄)alkyl, phenoxy, benzyloxy, benzoyloxy, cyano, isocyano, thiocyanato, isothiocyanato, nitro, -NR'''R'''', -NHCOR"', -NHCONR'''R"", -CONR'''R"", -SO₂R''', -OSO₂R''', -COR''', -CR'''=NR"" or -N=CR'''R'''', in which R"' and R"" are independently hydrogen, C₁₋₄ alkyl, halo(C₁₋₄)alkyl, C₁₋₄ alkoxy, halo-(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy;
provided that Y is not CCH₃ when W is CH, X and Z are N, R is NHCH₃, R¹ is 2,6-dichlorophenyl and R² is H.
Of particular interest are compounds where R⁸ is H.

The invention includes a compound of the general formula (1) as defined immediately above except that: C₁₋₈ alkoxy and C₁₋₈ alkylthio are excluded as values of R and R²; C₇ alkylene and C₃₋₇ alkenylene are excluded as chains formed by R³ and R⁴; the C₃₋₆ chain that R³ and R⁴ may form may only be optionally substituted with one or more methyl groups; thiomorpholine, thiomorpholine S-oxide, thiomorpholine S-dioxide and piperazine are excluded as rings that R³ and R⁴ may form; tri(C₁₋₄)alkylsilyl is excluded as a substituent of any alkyl, alkenyl, alkynyl or cycloalkyl group or moiety, and the morpholine ring that R³ and R⁴ may form is unsubstituted.

In still yet another aspect the invention provides a compound of the general formula (1) wherein
W and Y are both N and X and Z are both CR⁸ or X and Z are both N and W and Y are both CR⁸;
R⁸ is H, halo (e.g. fluoro, chloro or bromo), C₁₋₄ alkyl (e.g. methyl), C₁₋₄ alkoxy (e.g. methoxy) or halo(C₁₋₄)alkyl (e.g. trifluoromethyl);
R is H, halo (e.g. fluoro, chloro or bromo), C₁₋₄ alkyl (e.g. methyl), C₁₋₄ alkoxy (e.g. methoxy) or cyano;
R¹ is phenyl optionally substituted with from one to five halogen atoms or with from one to three substituents selected from halo, C₁₋₄ alkyl, halo(C₁₋₄)alkyl, C₁₋₄ alkoxy or halo(C₁₋₄)-alkoxy, pyridyl optionally substituted with from one to four halogen atoms or with from one to three substituents selected from halo, C₁₋₄ alkyl, halo(C₁₋₄)alkyl, C₁₋₄ alkoxy or halo(C₁₋₄)-alkoxy, 2- or 3-thienyl optionally substituted with from one to three halogen atoms or with from one to three substituents selected from halo, C₁₋₄ alkyl, halo(C₁₋₄)alkyl, C₁₋₄ alkoxy or halo(C₁₋₄)alkoxy, or piperidino or morpholino both optionally substituted with one or two methyl groups;
R² is NR³R⁴;
R³ is C₁₋₈ alkyl, halo(C₁₋₈)alkyl, hydroxy(C₁₋₈)alkyl, C₁₋₄ alkoxy(C₁₋₈)alkyl, C₁₋₄ alkoxyhalo-(C₁₋₈)alkyl, tri(C₁₋₄)alkylsilyl(C₁₋₆)alkyl, C₁₋₄ alkylcarbonyl(C₁₋₈)alkyl, C₁₋₄ alkylcarbonylhalo(C₁₋₈)alkyl, phenyl(₁₋₄)alkyl, C₂₋₈ alkenyl, halo(C₂₋₈)alkenyl, C₂₋₈ alkynyl, C₃₋₈ cycloalkyl optionally substituted with chloro, fluoro or methyl, C₃₋₈ cycloalkyl(C₁₋₄)alkyl, phenylamino, piperidino or morpholino, the phenyl ring of phenylalkyl or phenylamino being optionally substituted with one, two or three substituents selected from halo, C₁₋₄ alkyl, halo(C₁₋₄)alkyl, C₁₋₄ alkoxy and halo(C₁₋₄)alkoxy; and
R⁴ is H, C₁₋₄ alkyl, halo(C₁₋₄)alkyl or amino, or
R³ and R⁴ together form a C₃₋₇ alkylene or C₃₋₇ alkenylene chain optionally substituted with methyl, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring, in which the morpholine or piperazine rings are optionally substituted with methyl;
provided that Y is not CCH₃ when W is CH, X and Z are N, R is NHCH₃, R¹ is 2,6-dichlorophenyl and R² is H.
Of particular interest are compounds where R⁸ is H.
In still yet another aspect the invention provides a compound of the general formula (1) wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
R is halo;
R¹ is phenyl optionally substituted with from one to five halogen atoms or with from one to three substituents selected from halo, C₁₋₄ alkyl, halo(C₁₋₄)alkyl; C₁₋₄ alkoxy or halo(C₁₋₄)-alkoxy;
R² is NR³R⁴;
R³ is C₁₋₄ alkyl, halo(C₁₋₄)alkyl, C₂₋₄ alkenyl, C₃₋₆ cycloalkyl, C₃₋₆ cycloalkyl(C₁₋₄)alkyl or phenylamino in which the phenyl ring is optionally substituted with one, two or three substituents selected from halo, C₁₋₄ alkyl, halo(C₁₋₄)alkyl, C₁₋₄ alkoxy and halo(C₁₋₄)alkoxy; and
R⁴ is H, C₁₋₄ alkyl or amino, or R³ and R⁴ together form a C₄₋₆ alkylene chain optionally substituted with methyl, or, together with the nitrogen atom to which they are attached, R³ and R⁴ form a morpholine ring.
Of particular interest are compounds where R⁸ is H.

Compounds that form part of the invention are illustrated in Tables 1 to 126 below. Characterising data are given later in the Examples and in Table 132.

In Table 1 the compounds have the general formula (1A), where W and Y are N, X and Z are CH, R is Cl, R¹ is 2,4,6-trifluorophenyl and R³ and R⁴ are as shown in the table.

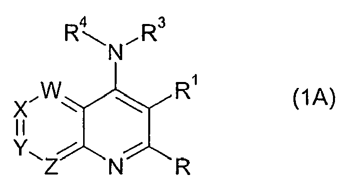

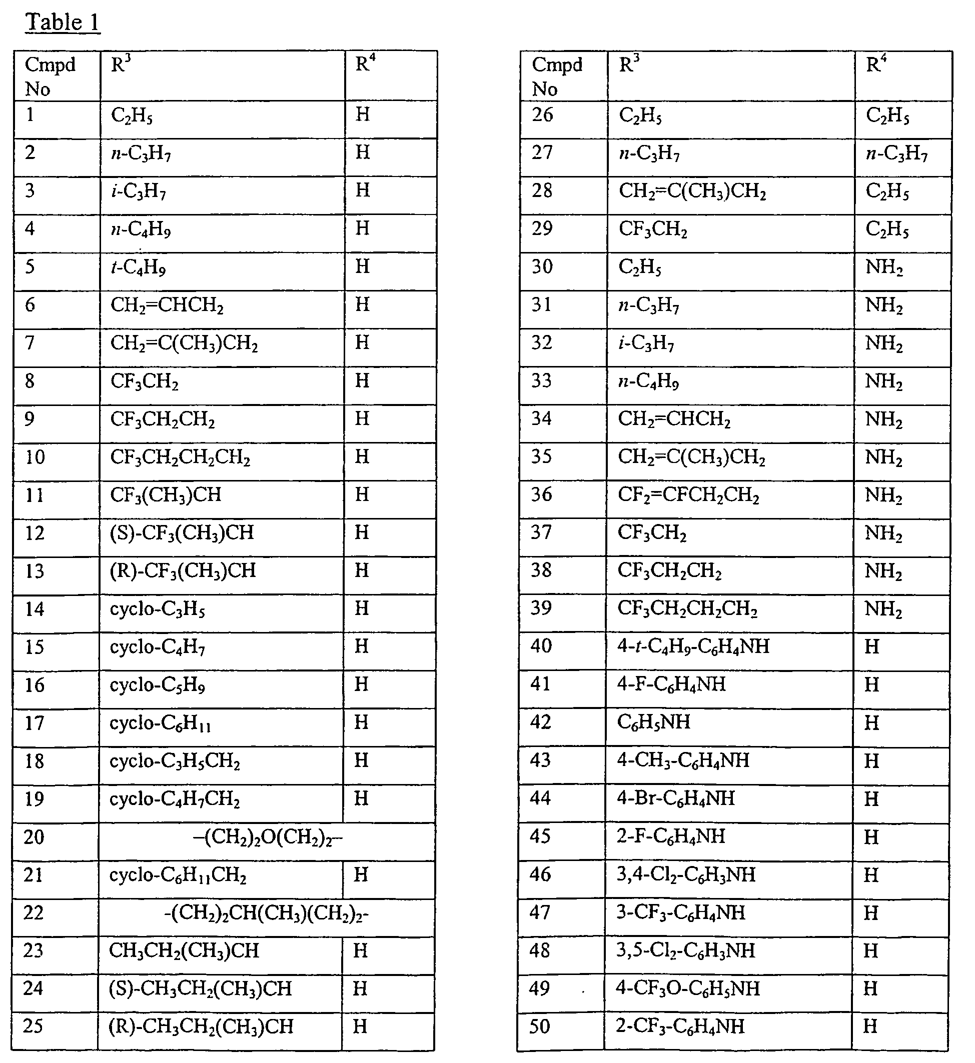

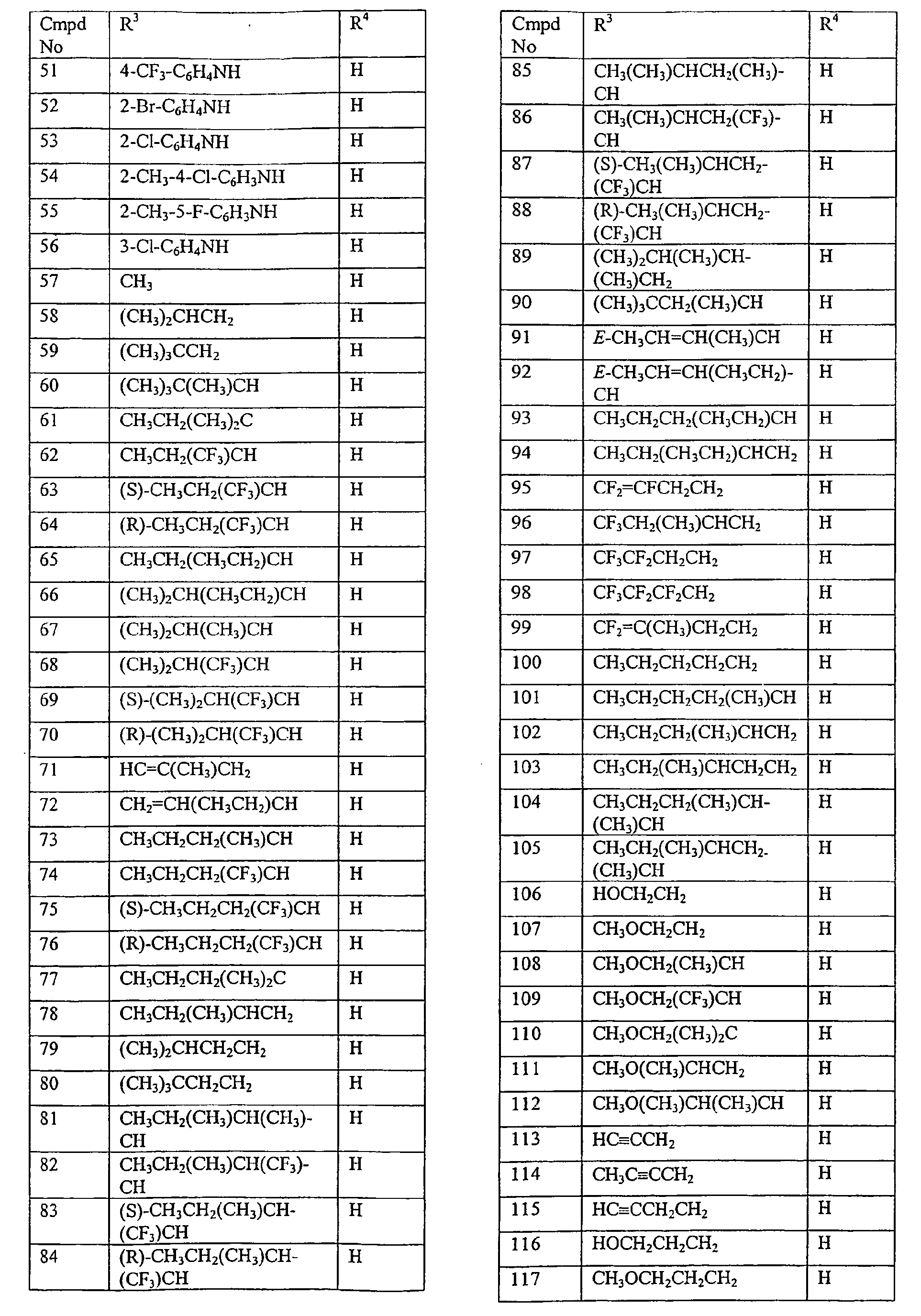

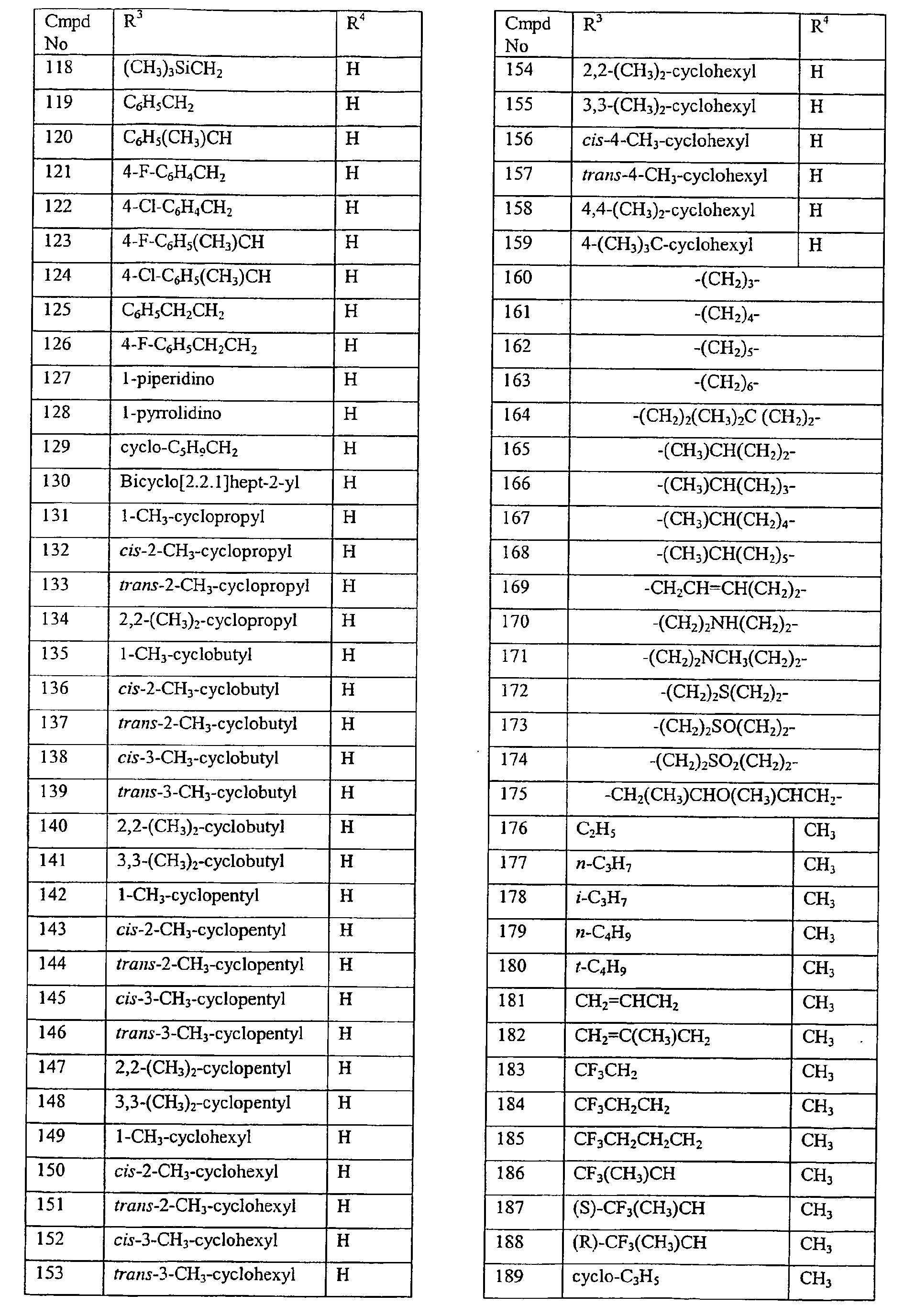

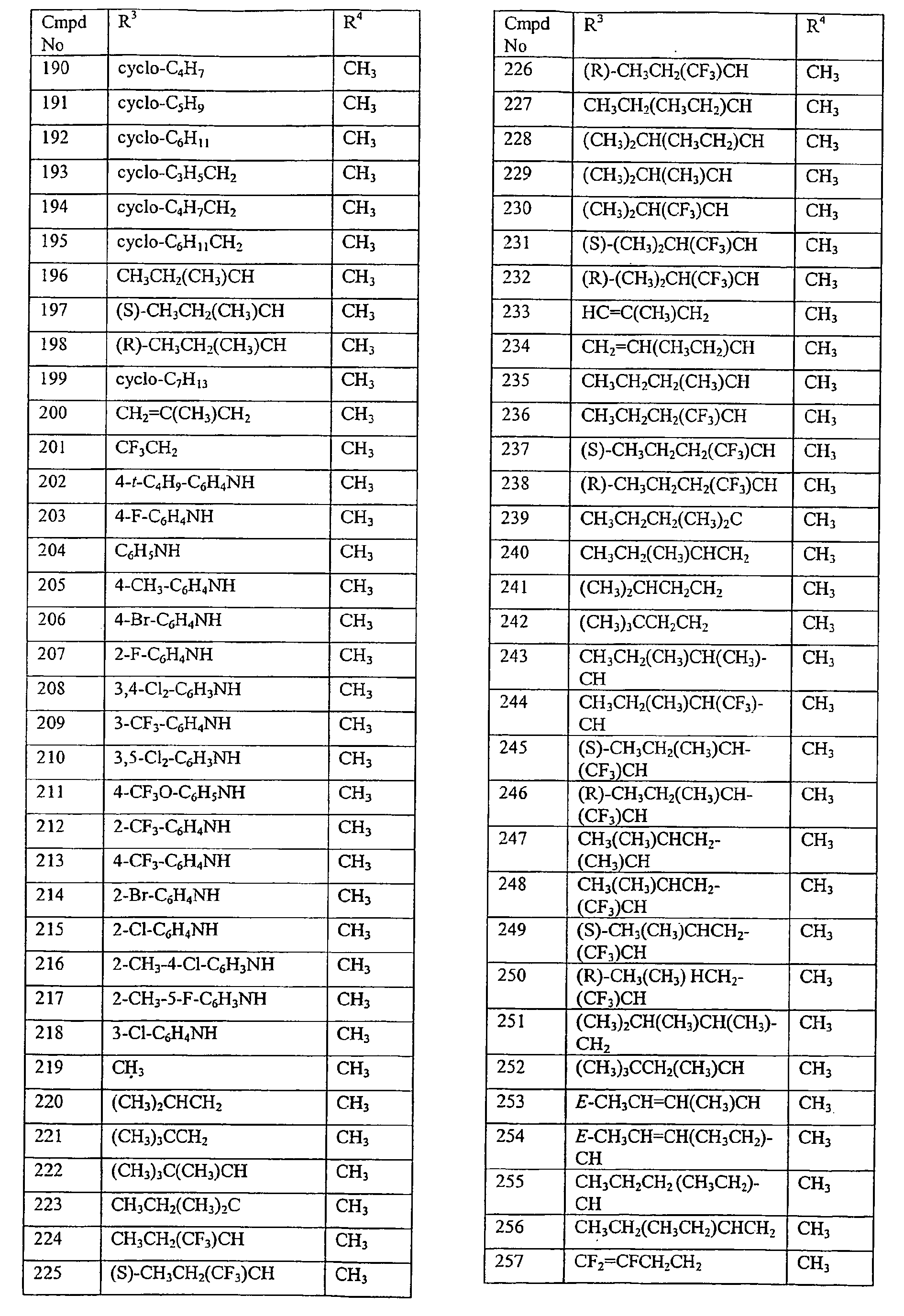

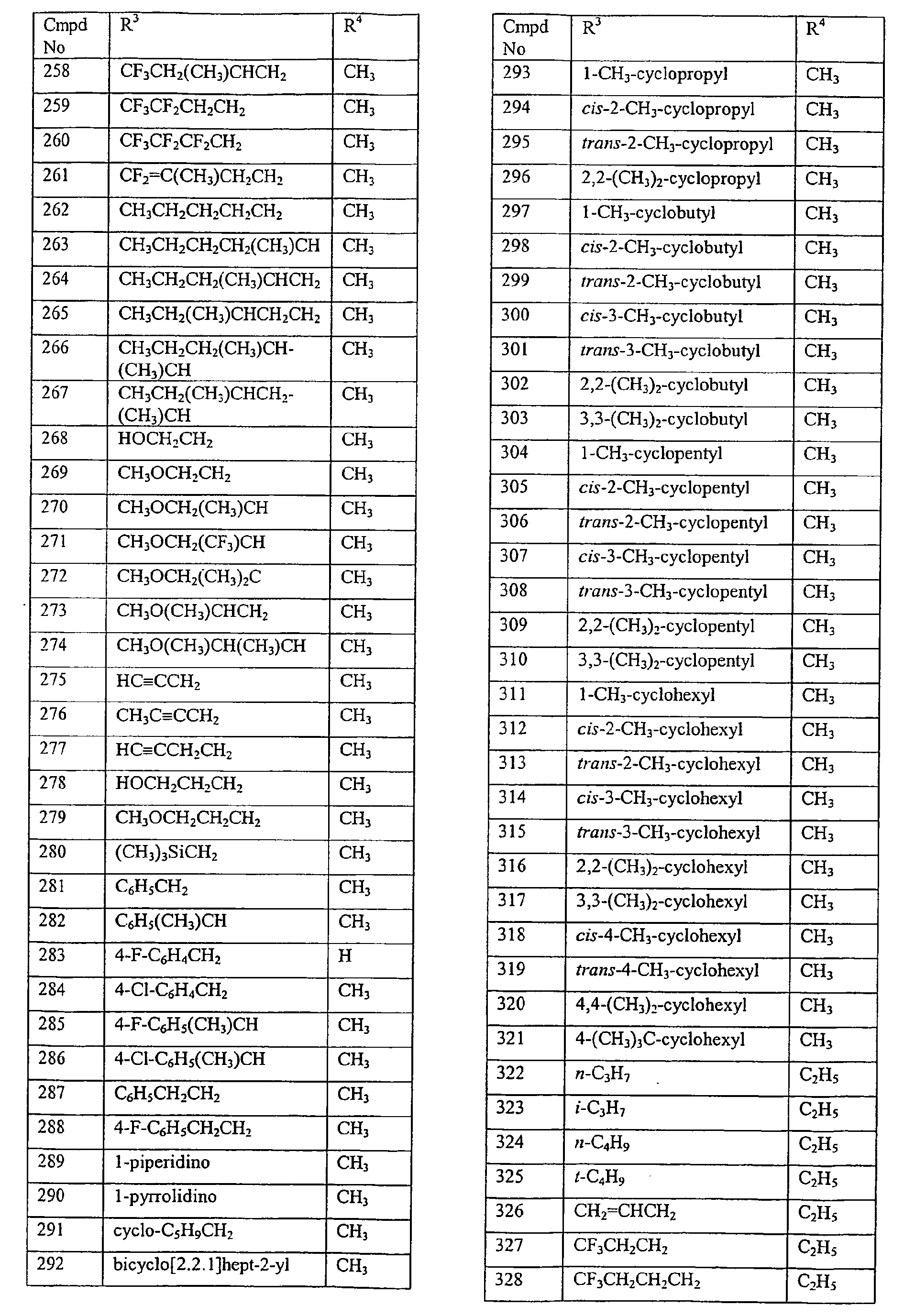

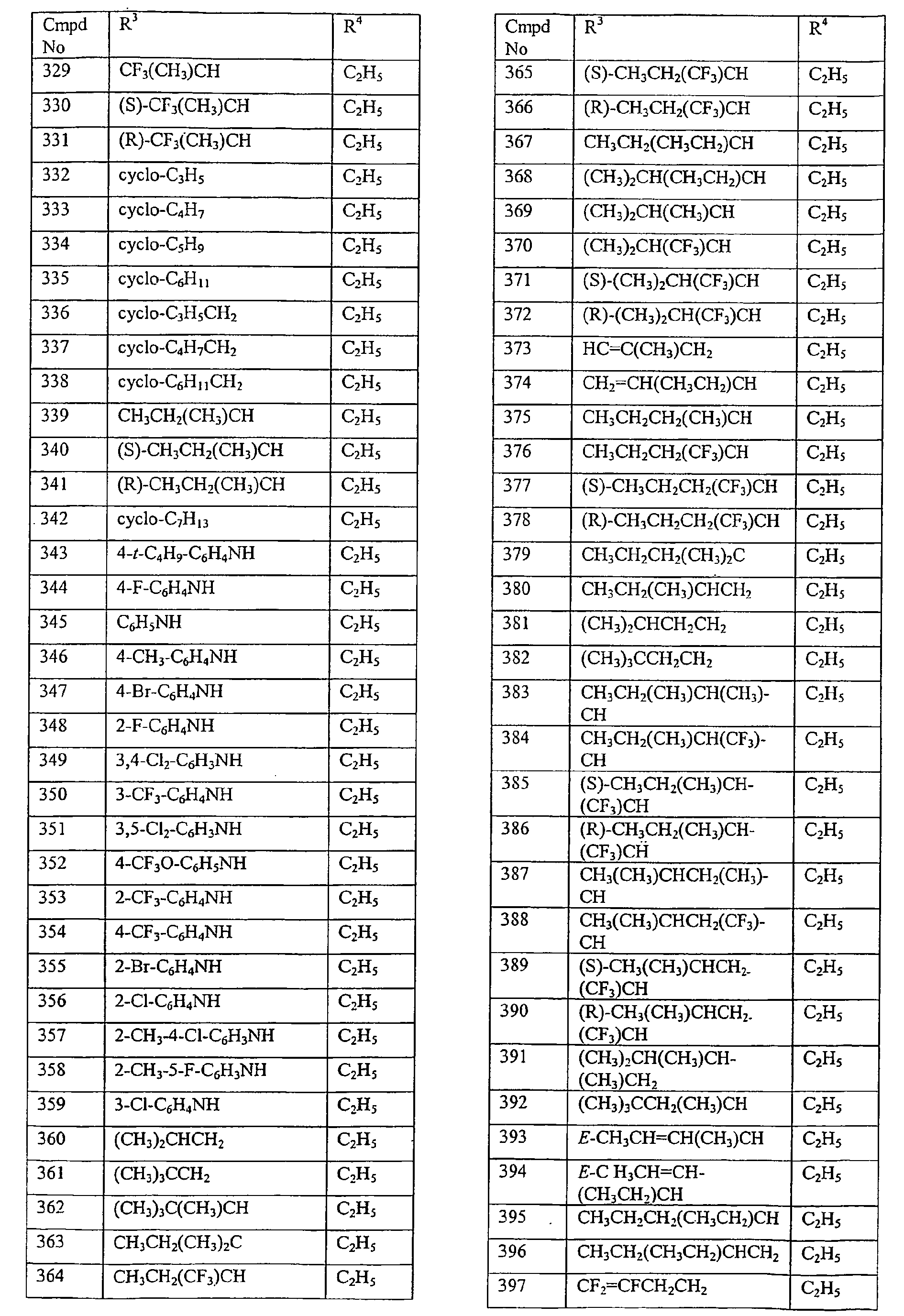

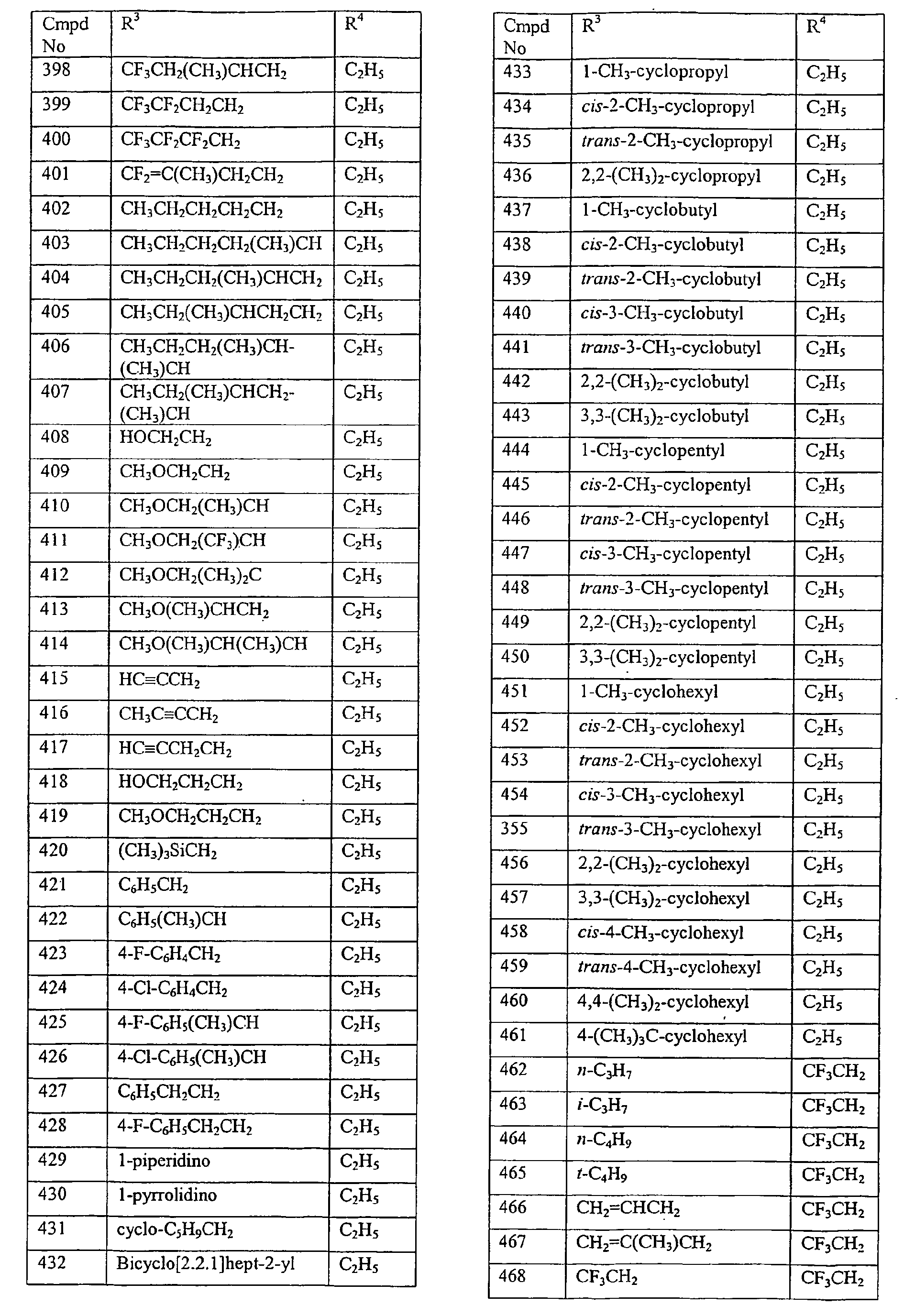

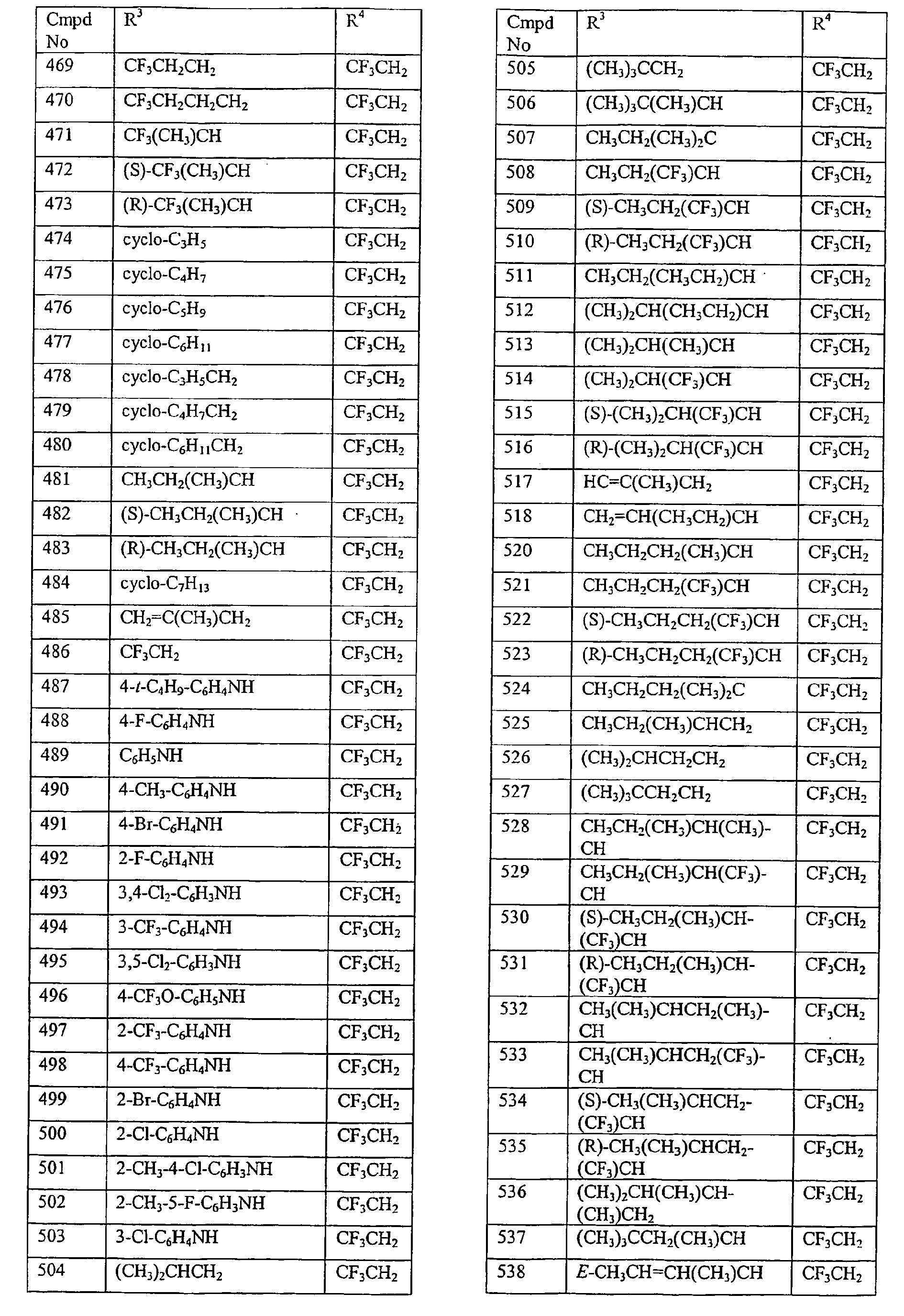

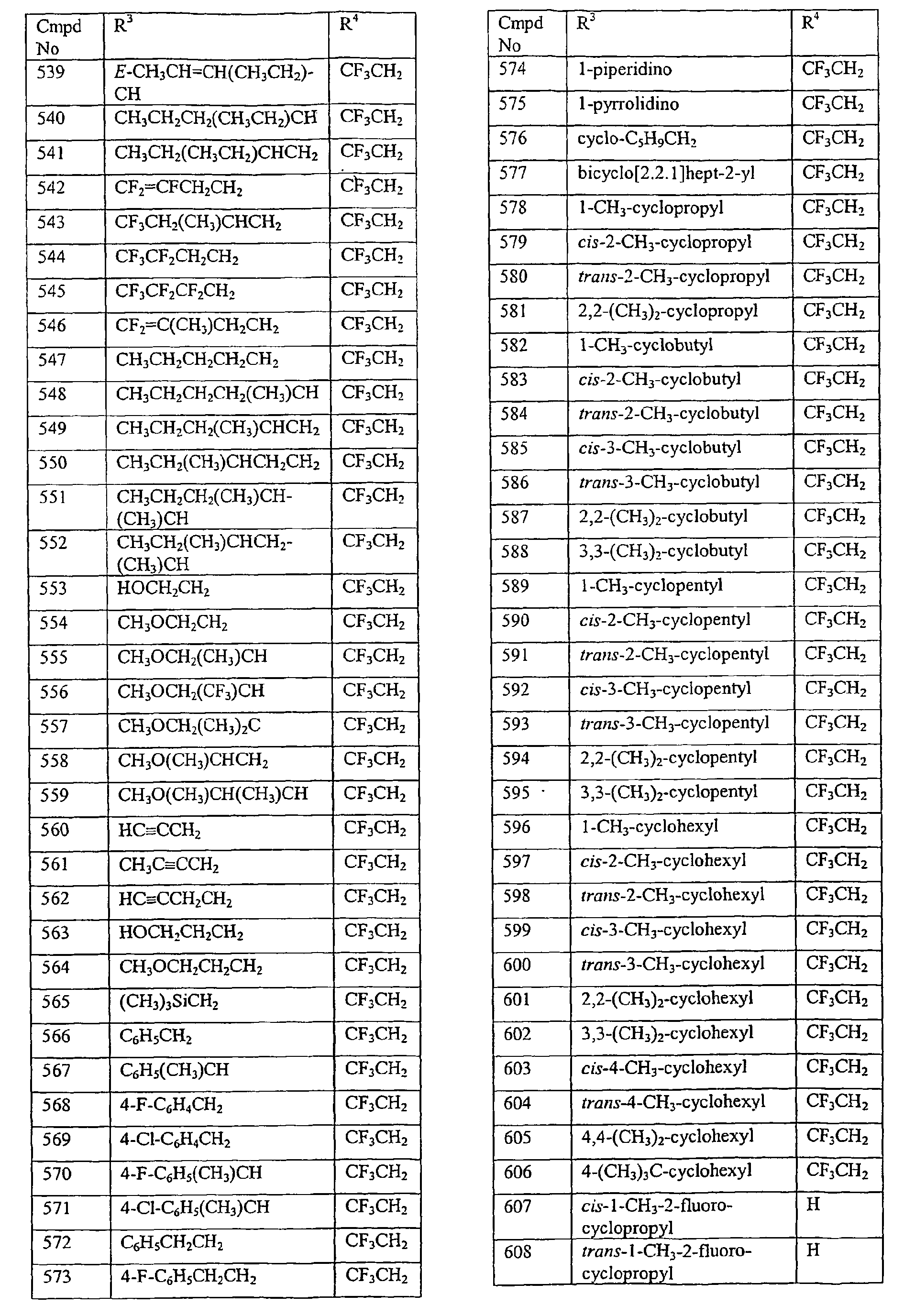

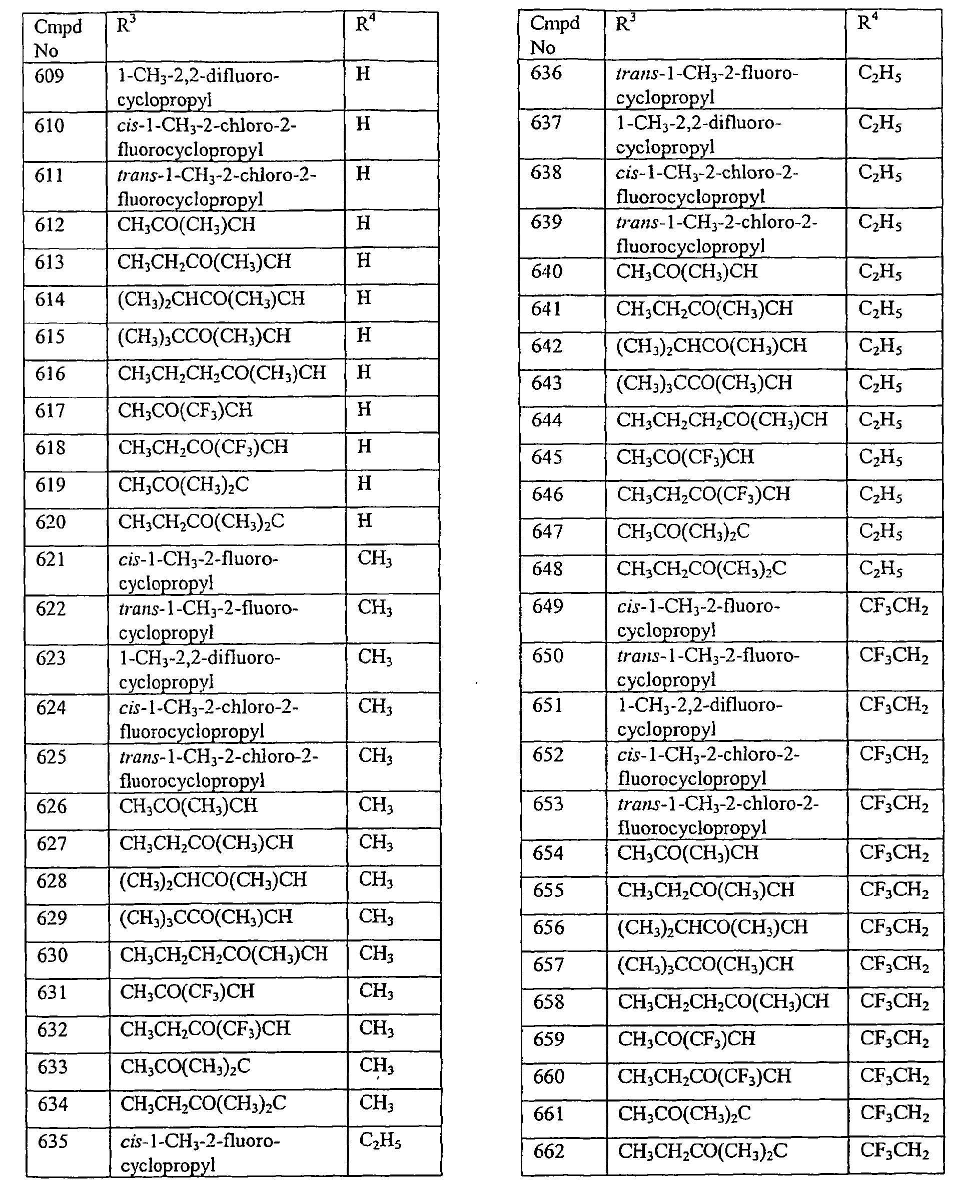

### Table 2

Table 2 consists of 662 compounds of the general formula (1A), where W and Y are N, X and Z are CH, R is Cl, R¹ is 2,5,6-trifluorophenyl, and the values of R³ and R⁴ are as listed in Table 1. Thus, compound 1 of Table 2 is the same as compound 1 of Table 1 except that in compound 1 of Table 2, R¹ is 2,5,6-trifluorophenyl. Similarly, compounds 2 to 662 of Table 2 are the same as compounds 2 to 662 of Table 1 except that in the compounds of Table 2, R¹ is 2,5,6-trifluorophenyl.

### Table 3

Table 3 consists of 662 compounds of the general formula (1A), where W and Y are N, X and Z are CH, R is Cl, R¹ is 2,3,4,5,6-pentafluorophenyl, and the values of R³ and R⁴ are as listed in Table 1. Thus, compound 1 of Table 3 is the same as compound 1 of Table 1 except that in compound 1 of Table 3, R¹ is 2,3,4,5,6-pentafluorophenyl. Similarly, compounds 2 to 662 of Table 3 are the same as compounds 2 to 662 of Table 1 except that in the compounds of Table 3, R¹ is 2,3,4,5,6-pentafluorophenyl.

### Table 4

Table 4 consists of 662 compounds of the general formula (1A), where W and Y are N, X and Z are CH, R is Cl, R¹ is 2,6-difluoro-4-methoxyphenyl, and the values of R³ and R⁴ are as listed in Table 1. Thus, compound 1 of Table 4 is the same as compound 1 of Table 1 except that in compound 1 of Table 4, R¹ is 2,6-difluoro-4-methoxyphenyl. Similarly, compounds 2 to 662 of Table 4 are the same as compounds 2 to 662 of Table 1 except that in the compounds of Table 4, R¹ is 2,6-difluoro-4-methoxyphenyl.

### Table 5

Table 5 consists of 662 compounds of the general formula (1A), where W and Y are N, X and Z are CH, R is Cl, R¹ is 2-fluoro-6-chlorophenyl, and the values of R³ and R⁴ are as listed in Table 1. Thus, compound 1 of Table 5 is the same as compound 1 of Table 1 except that in compound 1 of Table 5, R¹ is 2-fluoro-6-chlorophenyl. Similarly, compounds 2 to 662 of Table 5 are the same as compounds 2 to 662 of Table 1 except that in the compounds of Table 5, R¹ is 2-fluoro-6-chlorophenyl.

### Table 6

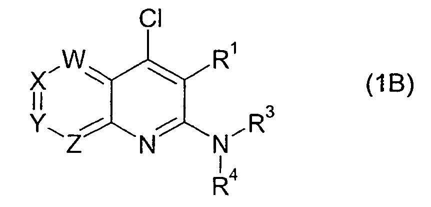

Table 6 consists of 662 compounds of the general formula (1B), where W and Y are N, X and Z are CH, R is Cl, R¹ is 2,4,6-trifluorophenyl, and the values of R³ and R⁴ are as listed in Table 1. Thus, compound 1 of Table 6 is the same as compound 1 of Table 1 except that in compound 1 of Table 6, the compound has the general formula (1B). Similarly, compounds 2 to 662 of Table 6 are the same as compounds 2 to 662 of Table 1 except that in the compounds of Table 6, the compounds have the general formula (1B).

### Table 7

Table 7 consists of 662 compounds of the general formula (1B), where W and Y are N, X and Z are CH, R is Cl, R¹ is 2,5,6-trifluorophenyl, and the values of R³ and R⁴ are as listed in Table 1. Thus, compound 1 of Table 7 is the same as compound 1 of Table 2 except that in compound 1 of Table 7, the compound has the general formula (1B). Similarly, compounds 2 to 662 of Table 7 are the same as compounds 2 to 662 of Table 2 except that in the compounds of Table 7, the compounds have the general formula (1B).

### Table 8

Table 8 consists of 662 compounds of the general formula (1B), where W and Y are N, X and Z are CH, R is Cl, R¹ is 2,3,4,5,6-pentafluorophenyl, and the values of R³ and R⁴ are as listed in Table 3. Thus, compound 1 of Table 8 is the same as compound 1 of Table 3 except that in compound 1 of Table 8, the compound has the general formula (1B). Similarly, compounds 2 to 662 of Table 8 are the same as compounds 2 to 662 of Table 3 except that in the compounds of Table 8, the compounds have the general formula (1B).

### Table 9

Table 9 consists of 662 compounds of the general formula (1B), where W and Y are N, X and Z are CH, R is Cl, R¹ is 2,6-difluoro-4-methoxyphenyl, and the values of R³ and R⁴ are as listed in Table 1. Thus, compound 1 of Table 9 is the same as compound 1 of Table 4 except that in compound 1 of Table 9, the compound has the general formula (1B). Similarly, compounds 2 to 662 of Table 9 are the same as compounds 2 to 662 of Table 4 except that in the compounds of Table 9, the compounds have the general formula (1B).

### Table 10

Table 10 consists of 662 compounds of the general formula (1B), where W and Y are N, X and Z are CH, R is Cl, R¹ is 2-fluoro-6-chlorophenyl, and the values of R³ and R⁴ are as listed in Table 1. Thus, compound 1 of Table 10 is the same as compound 1 of Table 5 except that in compound 1 of Table 10, the compound has the general formula (1B). Similarly, compounds 2 to 662 of Table 10 are the same as compounds 2 to 662 of Table 5 except that in the compounds of Table 10, the compounds have the general formula (1B).

### Table 11

Table 11 consists of 662 compounds of the general formula (1A), where W and Y are CH, X and Z are N, R is Cl, R¹ is 2,4,6-trifluorophenyl, and the values of R³ and R⁴ are as listed in Table 1. Thus, compound 1 of Table 11 is the same as compound 1 of Table 1 except that in compound 1 of Table 11, the compound has the general formula (1A) where W and Y are CH, X and Z are N. Similarly, compounds 2 to 662 of Table 11 are the same as compounds 2 to 662 of Table 1 except that in the compounds of Table 11, the compounds have the general formula (1A) where W and Y are CH, X and Z are N.

### Table 12

Table 12 consists of 662 compounds of the general formula (1A), where W and Y are CH, X and Z are N, R is Cl, R¹ is 2,5,6-trifluorophenyl, and the values of R³ and R⁴ are as listed in Table 1. Thus, compound 1 of Table 12 is the same as compound 1 of Table 2 except that in compound 1 of Table 12, the compound has the general formula (1A) where W and Y are CH, X and Z are N. Similarly, compounds 2 to 662 of Table 12 are the same as compounds 2 to 662 of Table 2 except that in the compounds of Table 12, the compounds have the general formula (1A) where W and Y are CH, X and Z are N.

### Table 13

Table 13 consists of 662 compounds of the general formula (1A), where W and Y are CH, X and Z are N, R is Cl, R¹ is 2,3,4,5,6-pentafluorophenyl, and the values of R³ and R⁴ are as listed in Table 1. Thus, compound 1 of Table 13 is the same as compound 1 of Table 3 except that in compound 1 of Table 13, the compound has the general formula (1A) where W and Y are CH, X and Z are N. Similarly, compounds 2 to 662 of Table 13 are the same as compounds 2 to 662 of Table 3 except that in the compounds of Table 13, the compounds have the general formula (1A) where W and Y are CH, X and Z are N.

### Table 14

Table 14 consists of 662 compounds of the general formula (1A), where W and Y are CH, X and Z are N, R is Cl, R¹ is 2,6-difluoro-4-methoxyphenyl, and the values of R³ and R⁴ are as listed in Table 1. Thus, compound 1 of Table 14 is the same as compound 1 of Table 4 except that in compound 1 of Table 14, the compound has the general formula (1A) where W and Y are CH, X and Z are N. Similarly, compounds 2 to 662 of Table 14 are the same as compounds 2 to 662 of Table 4 except that in the compounds of Table 14, the compounds have the general formula (1A) where W and Y are CH, X and Z are N.

### Table 15

Table 1 consists of 662 compounds of the general formula (1A), where W and Y are CH, X and Z are N, R is Cl, R¹ is 2-fluoro-6-chlorophenyl, and the values of R³ and R⁴ are as listed in Table 1. Thus, compound 1 of Table 15 is the same as compound 1 of Table 5 except that in compound 1 of Table 15, the compound has the general formula (1A) where W and Y are CH, X and Z are N. Similarly, compounds 2 to 662 of Table 15 are the same as compounds 2 to 662 of Table 5 except that in the compounds of Table 15, the compounds have the general formula (1A) where W and Y are CH, X and Z are N.

### Table 16

Table 16 consists of 662 compounds of the general formula (1B), where W and Y are CH, X and Z are N, R is Cl, R¹ is 2,4,6-trifluorophenyl, and the values of R³ and R⁴ are as listed in Table 1. Thus, compound 1 of Table 16 is the same as compound 1 of Table 11 except that in compound 1 of Table 16, the compound has the general formula (1B). Similarly, compounds 2 to 662 of Table 16 are the same as compounds 2 to 662 of Table 11 except that in the compounds of Table 16, the compounds have the general formula (1B).

### Table 17

Table 17 consists of 662 compounds of the general formula (1B), where W and Y are CH, X and Z are N, R is Cl, R¹ is 2,5,6-trifluorophenyl, and the values of R³ and R⁴ are as listed in Table 1. Thus, compound 1 of Table 17 is the same as compound 1 of Table 12 except that in compound 1 of Table 17, the compound has the general formula (1B). Similarly, compounds 2 to 662 of Table 17 are the same as compounds 2 to 662 of Table 12 except that in the compounds of Table 17, the compounds have the general formula (1B).

### Table 18

Table 18 consists of 662 compounds of the general formula (1B), where W and Y are CH, X and Z are N, R is Cl, R¹ is 2,3,4,5,6-pentafluorophenyl, and the values of R³ and R⁴ are as listed in Table 1. Thus, compound 1 of Table 18 is the same as compound 1 of Table 13 except that in compound 1 of Table 18, the compound has the general formula (1B). Similarly, compounds 2 to 662 of Table 18 are the same as compounds 2 to 662 of Table 13 except that in the compounds of Table 18, the compounds have the general formula (1B).

### Table 19

Table 19 consists of 662 compounds of the general formula (1B), where W and Y are CH, X and Z are N, R is Cl, R¹ is 2,6-difluoro-4-methoxyphenyl, and the values of R³ and R⁴ are as listed in Table 1. Thus, compound 1 of Table 19 is the same as compound 1 of Table 14 except that in compound 1 of Table 19, the compound has the general formula (1B). Similarly, compounds 2 to 662 of Table 19 are the same as compounds 2 to 662 of Table 14 except that in the compounds of Table 19, the compounds have the general formula (1B).

### Table 20

Table 20 consists of 662 compounds of the general formula (1B), where W and Y are CH, X and Z are N, R is Cl, R¹ is 2-fluoro-6-chlorophenyl, and the values of R³ and R⁴ are as listed in Table 1. Thus, compound 1 of Table 20 is the same as compound 1 of Table 15 except that in compound 1 of Table 20, the compound has the general formula (1B). Similarly, compounds 2 to 662 of Table 20 are the same as compounds 2 to 662 of Table 15 except that in the compounds of Table 20, the compounds have the general formula (1B).

### Table 21

Table 21 consists of 662 compounds of the general formula (1A), where W and Y are N and X and Z are CH, R is F, R¹ is 2,4,6-trifluorophenyl, and the values of R³ and R⁴ are as listed in Table 1. Thus, compound 1 of Table 21 is the same as compound 1 of Table 1 except that in compound 1 of Table 21, R is F instead of Cl. Similarly, compounds 2 to 662 of Table 21 are the same as compounds 2 to 662 of Table 1 except that in the compounds of Table 21, R is F instead of Cl.

### Table 22

Table 22 consists of 662 compounds of the general formula (1A), where W and Y are N and X and Z are CH, R is F, R¹ is 2,5,6-trifluorophenyl, and the values of R³ and R⁴ are as listed in Table 1. Thus, compound 1 of Table 22 is the same as compound 1 of Table 2 except that in compound 1 of Table 22, R is F instead of Cl. Similarly, compounds 2 to 662 of Table 22 are the same as compounds 2 to 662 of Table 2 except that in the compounds of Table 22, R is F instead of Cl.

### Table 23

Table 23 consists of 662 compounds of the general formula (1A), where W and Y are N and X and Z are CH, R is F, R¹ is 2,3,4,5,6-pentafluorophenyl, and the values of R³ and R⁴ are as listed in Table 1. Thus, compound 1 of Table 23 is the same as compound 1 of Table 3 except that in compound 1 of Table 23, R is F instead of Cl. Similarly, compounds 2 to 662 of Table 23 are the same as compounds 2 to 662 of Table 3 except that in the compounds of Table 23, R is F instead of Cl.

### Table 24

Table 24 consists of 662 compounds of the general formula (1A), where W and Y are N and X and Z are CH, R is F, R¹ is 2,6-difluoro-4-methoxyphenyl, and the values of R³ and R⁴ are as listed in Table 1. Thus, compound 1 of Table 24 is the same as compound 1 of Table 4 except that in compound 1 of Table 24, R is F instead of Cl. Similarly, compounds 2 to 662 of Table 24 are the same as compounds 2 to 662 of Table 4 except that in the compounds of Table 24, R is F instead of Cl.

### Table 25

Table 25 consists of 662 compounds of the general formula (1A), where W and Y are N and X and Z are CH, R is F, R¹ is 2-fluoro-6-chlorophenyl, and the values of R³ and R⁴ are as listed in Table 1. Thus, compound 1 of Table 25 is the same as compound 1 of Table 5 except that in compound 1 of Table 25, R is F instead of Cl. Similarly, compounds 2 to 662 of Table 25 are the same as compounds 2 to 662 of Table 5 except that in the compounds of Table 25, R is F instead of Cl.

### Table 26

Table 26 consists of 662 compounds of the general formula (1A), where W and Y are CH and X and Z are N, R is F, R¹ is 2,4,6-trifluorophenyl, and the values of R³ and R⁴ are as listed in Table 1. Thus, compound 1 of Table 26 is the same as compound 1 of Table 11 except that in compound 1 of Table 26, R is F instead of Cl. Similarly, compounds 2 to 662 of Table 26 are the same as compounds 2 to 662 of Table 11 except that in the compounds of Table 26, R is F instead of Cl.

### Table 27

Table 27 consists of 662 compounds of the general formula (1A), where W and Y are CH and X and Z are N, R is F, R¹ is 2,5,6-trifluorophenyl, and the values of R³ and R⁴ are as listed in Table 1. Thus, compound 1 of Table 27 is the same as compound 1 of Table 12 except that in compound 1 of Table 27, R is F instead of Cl. Similarly, compounds 2 to 662 of Table 27 are the same as compounds 2 to 662 of Table 12 except that in the compounds of Table 27, R is F instead of Cl.

### Table 28

Table 28 consists of 662 compounds of the general formula (1A), where W and Y are CH and X and Z are N, R is F, R¹ is 2,3,4,5,6-pentafluorophenyl, and the values of R³ and R⁴ are as listed in Table 1. Thus, compound 1 of Table 28 is the same as compound 1 of Table 13 except that in compound 1 of Table 28, R is F instead of Cl. Similarly, compounds 2 to 662 of Table 28 are the same as compounds 2 to 662 of Table 13 except that in the compounds of Table 28, R is F instead of Cl.

### Table 29

Table 29 consists of 662 compounds of the general formula (1A), where W and Y are CH and X and Z are N, R is F, R¹ is 2,6-difluoro-4-methoxyphenyl, and the values of R³ and R⁴ are as listed in Table 1. Thus, compound 1 of Table 29 is the same as compound 1 of Table 14 except that in compound 1 of Table 29, R is F instead of Cl. Similarly, compounds 2 to 662 of Table 29 are the same as compounds 2 to 662 of Table 14 except that in the compounds of Table 29, R is F instead of Cl.

### Table 30

Table 30 consists of 662 compounds of the general formula (1A), where W and Y are CH and X and Z are N, R is F, R¹ is 2-fluoro-6-chlorophenyl, and the values of R³ and R⁴ are as listed in Table 1. Thus, compound 1 of Table 30 is the same as compound 1 of Table 15 except that in compound 1 of Table 30, R is F instead of Cl. Similarly, compounds 2 to 662 of Table 30 are the same as compounds 2 to 662 of Table 15 except that in the compounds of Table 30, R is F instead of Cl.

### Table 31

Table 31 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 31 R¹ is 2,6-difluorophenyl instead of 2-fluoro-6-chlorophenyl.

### Table 32

Table 32 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 32 R¹ is 2-fluorophenyl instead of 2-fluoro-6-chlorophenyl.

### Table 33

Table 33 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 33 R¹ is 2,3,5,6-tetrafluorophenyl instead of 2-fluoro-6-chlorophenyl.

### Table 34

Table 34 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 34 R¹ is 2-chloro-4,6-difluorophenyl instead of 2-fluoro-6-chlorophenyl.

### Table 35

Table 35 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 35 R¹ is 2-chlorophenyl instead of 2-fluoro-6-chlorophenyl.

### Table 36

Table 36 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 36 R¹ is 2,6-dichlorophenyl instead of 2-fluoro-6-chlorophenyl.

### Table 37

Table 37 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 37 R¹ is 2,4-dichlorophenyl instead of 2-fluoro-6-chlorophenyl.

### Table 38

Table 38 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 38 R¹ is 2,4,6-trichlorophenyl instead of 2-fluoro-6-chlorophenyl.

### Table 39

Table 39 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 39 R¹ is 2,3,6-trichlorophenyl instead of 2-fluoro-6-chlorophenyl.

### Table 40

Table 40 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 40 R¹ is pentachlorophenyl instead of 2-fluoro-6-chlorophenyl.

### Table 41

Table 41 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 41 R¹ is 2-fluoro-4,6-dichlorophenyl instead of 2-fluoro-6-chlorophenyl.

### Table 42

Table 42 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 42 R¹ is 4-fluoro-2,6-dichlorophenyl instead of 2-fluoro-6-chlorophenyl.

### Table 43

Table 43 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 43 R¹ is 2-bromophenyl instead of 2-fluoro-6-chlorophenyl.

### Table 44

Table 44 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 44 R¹ is 2-fluoro-6-bromophenyl instead of 2-fluoro-6-chlorophenyl.

### Table 45

Table 45 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 45 R¹ is 2-bromo-4,6-difluorophenyl instead of 2-fluoro-6-chlorophenyl.

### Table 46

Table 46 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 46 R¹ is 2-fluoro-6-methylphenyl instead of 2-fluoro-6-chlorophenyl.

### Table 47

Table 47 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 47 R¹ is 2-chloro-6-methylphenyl instead of 2-fluoro-6-chlorophenyl.

### Table 48

Table 48 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 48 R¹ is 2-methoxyphenyl instead of 2-fluoro-6-chlorophenyl.

### Table 49

Table 49 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 49 R¹ is 2,6-dimethoxyphenyl instead of 2-fluoro-6-chlorophenyl.

### Table 50

Table 50 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 50 R¹ is 2-fluoro-6-methoxyphenyl instead of 2-fluoro-6-chlorophenyl.

### Table 51

Table 51 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 51 R¹ is 2-trifluoromethylphenyl instead of 2-fluoro-6-chlorophenyl.

### Table 52

Table 52 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 52 R¹ is 2-fluoro-6-trifluoromethylphenyl instead of 2-fluoro-6-chlorophenyl.

### Table 53

Table 53 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 53 R¹ is 2,6-di-(trifluoromethyl)phenyl instead of 2-fluoro-6-chlorophenyl.

### Table 54

Table 54 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 54 R¹ is 2-chloro-6-trifluoromethylphenyl instead of 2-fluoro-6-chlorophenyl.

### Table 55

Table 55 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 55 R¹ is 2,4-difluoro-6-trifluoromethylphenyl instead of 2-fluoro-6-chlorophenyl.

### Table 56

Table 56 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 56 R¹ is 2,4-difluoro-6-methoxyphenyl instead of 2-fluoro-6-chlorophenyl.

### Table 57

Table 57 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 57 R¹ is 2,4-difluoro-6-methylphenyl instead of 2-fluoro-6-chlorophenyl.

### Table 58

Table 58 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 58 R¹ is 2,4-difluoropyrid-3-yl instead of 2-fluoro-6-chlorophenyl.

### Table 59

Table 59 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 59 R¹ is 3,5-difluoropyrid-4-yl instead of 2-fluoro-6-chlorophenyl.

### Table 60

Table 60 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 60 R¹ is tetrafluoropyrid-4-yl instead of 2-fluoro-6-chlorophenyl.

### Table 61

Table 61 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 61 R¹ is 3-fluoropyrid-2-yl instead of 2-fluoro-6-chlorophenyl.

### Table 62

Table 62 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 62 R¹ is 4-fluoropyrid-3-yl instead of 2-fluoro-6-chlorophenyl.

### Table 63

Table 63 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 63 R¹ is 3-fluoropyrid-4-yl instead of 2-fluoro-6-chlorophenyl.

### Table 64

Table 64 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 64 R¹ is 2-fluoropyrid-3-yl instead of 2-fluoro-6-chlorophenyl.

### Table 65

Table 65 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 65 R¹ is 2,4,6-trifluoropyrid-3-yl instead of 2-fluoro-6-chlorophenyl.

### Table 66

Table 66 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 66 R¹ is 3,5-difluoropyrid-2-yl instead of 2-fluoro-6-chlorophenyl.

### Table 67

Table 67 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 67 R¹ is 2,6-difluoropyrid-3-yl instead of 2-fluoro-6-chlorophenyl.

### Table 68

Table 68 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 68 R¹ is 2,4-difluoro-6-methoxypyrid-3-yl instead of2-fluoro-6-chlorophenyl.

### Table 69

Table 69 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 69 R¹ is 2-fluoro-4-chloropyrid-3-yl instead of 2-fluoro-6-chlorophenyl.

### Table 70

Table 70 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 70 R¹ is 3-fluoro-5-chloropyrid-4-yl instead of 2-fluoro-6-chlorophenyl.

### Table 71

Table 71 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 71 R¹ is 2-chloro-4-fluoropyrid-3-yl instead of 2-fluoro-6-chlorophenyl.

### Table 72

Table 72 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 72 R¹ is 2,4-dichloropyrid-3-yl instead of 2-fluoro-6-chlorophenyl.

### Table 73

Table 73 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 73 R¹ is 3-chloropyrid-2-yl instead of 2-fluoro-6-chlorophenyl.

### Table 74

Table 74 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 74 R¹ is 4-chloropyrid-3-yl instead of 2-fluoro-6-chlorophenyl.

### Table 75

Table 75 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 75 R¹ is 3-chloropyrid-4-yl instead of 2-fluoro-6-chlorophenyl.

### Table 76

Table 76 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 76 R¹ is 2-chloropyrid-3-yl instead of 2-fluoro-6-chlorophenyl.

### Table 77

Table 77 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 77 R¹ is 3-trifluoromethylpyrid-2-yl instead of 2-fluoro-6-chlorophenyl.

### Table 78

Table 78 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 78 R¹ is 4-trifluoromethylpyrid-3-yl instead of 2-fluoro-6-chlorophenyl.

### Table 79

Table 79 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 79 R¹ is 3,5-dichloropyrid-2-yl instead of 2-fluoro-6-chlorophenyl.

### Table 80

Table 80 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 80 R¹ is 4,6-dichloropyrid-3-yl instead of 2-fluoro-6-chlorophenyl.

### Table 81

Table 81 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 81 R¹ is 3-trifluoromethylpyrid-4-yl instead of 2-fluoro-6-chlorophenyl.

### Table 82

Table 82 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 82 R¹ is 2-trifluoromethylpyrid-3-yl instead of 2-fluoro-6-chlorophenyl.

### Table 83

Table 83 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 83 R¹ is 2-fluoro-4-trifluoromethylpyrid-3-yl instead of 2-fluoro-6-chlorophenyl.

### Table 84

Table 84 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 84 R¹ is 3-fluoro-5-trifluoromethylpyrid-4-yl instead of 2-fluoro-6-chlorophenyl.

### Table 85

Table 85 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 85 R¹ is 4-fluoro-2-trifluoromethylpyrid-3-yl instead of 2-fluoro-6-chlorophenyl.

### Table 86

Table 86 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 86 R¹ is 2,6-dichloropyrid-3-yl instead of 2-fluoro-6-chlorophenyl.

### Table 87

Table 87 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 87 R¹ is 3,5-dichloropyrid-4-yl instead of 2-fluoro-6-chlorophenyl.

### Table 88

Table 88 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 88 R¹ is 3-chloro-6-trifluoromethylpyrid-2-yl instead of 2-fluoro-6-chlorophenyl.

### Table 89

Table 89 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 89 R¹ is 3-fluoro-6-trifluoromethylpyrid-2-yl instead of 2-fluoro-6-chlorophenyl.

### Table 90

Table 90 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 90 R¹ is pyrid-2-yl instead of 2-fluoro-6-chlorophenyl.

### Table 91

Table 91 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 91 R¹ is pyrid-3-yl instead of 2-fluoro-6-chlorophenyl.

### Table 92

Table 92 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 92 R¹ is pyrid-4-yl instead of 2-fluoro-6-chlorophenyl.

### Table 93

Table 93 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 93 R¹ is 3-fluorothien-2-yl instead of 2-fluoro-6-chlorophenyl.

### Table 94

Table 94 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 94 R¹ is 3-chlorothien-2-yl instead of 2-fluoro-6-chlorophenyl.

### Table 95

Table 95 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 95 R¹ is 2,4-difluorothien-3-yl instead of 2-fluoro-6-chlorophenyl.

### Table 96

Table 96 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 96 R¹ is 2,4-dichlorothien-3-yl instead of 2-fluoro-6-chlorophenyl.

### Table 97

Table 97 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 97 R¹ is 2,4,5-trichlorothien-3-yl instead of 2-fluoro-6-chlorophenyl.

### Table 98

Table 98 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 98 R¹ is piperidino instead of 2-fluoro-6-chlorophenyl.

### Table 99

Table 99 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 99 R¹ is 2-methylpiperidino instead of 2-fluoro-6-chlorophenyl.

### Table 100

Table 100 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 100 R¹ is 2,6-dimethylpiperidino instead of 2-fluoro-6-chlorophenyl.

### Table 101

Table 101 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 101 R¹ is morpholino instead of 2-fluoro-6-chlorophenyl.

### Table 102

Table 102 consists of 3972 compounds. Compounds 1 to 662 are exactly the same as compounds 1 to 662 of Table 5 respectively, compounds 663 to 1324 are exactly the same as compounds 1 to 662 of Table 10 respectively, compounds 1325 to 1986 are exactly the same as compounds 1 to 662 of Table 15 respectively, compounds 1987 to 2648 are exactly the same as compounds 1 to 662 of Table 20 respectively, compounds 2649 to 3310 are exactly the same as compounds 1 to 662 of Table 25 respectively, and compounds 3311 to 3972 are exactly the same as compounds 1 to 662 of Table 30 respectively, except that in all of the compounds of Table 102 R¹ is 2,6-dimethylmorpholino instead of 2-fluoro-6-chlorophenyl.

### Table 103

Table 103 consists of 305,844 compounds. Each of these compounds is exactly the same as the corresponding compound in Tables 1 to 102 (thus, for example, compound 1 of Table 103 is the same as compound 1 of Table 1, compound 663 of Table 103 is the same as compound 1 of Table 2, compound 19,861 of Table 103 is the same as compound 1 of Table 31, compound 305,844 of Table 103 is the same as compound 3,972 of Table 102) except that in all of the compounds of Table 103 R is ethyl instead of Cl or F.

### Table 104

Table 104 consists of 305,844 compounds. Each of these compounds is exactly the same as the corresponding compound in Tables 1 to 102 (thus, for example, compound 1 of Table 104 is the same as compound 1 of Table 1, compound 663 of Table 104 is the same as compound 1 of Table 2, compound 19,861 of Table 104 is the same as compound 1 of Table 31, compound 305,844 of Table 104 is the same as compound 3,972 of Table 102) except that in all of the compounds of Table 104 R is Br instead of Cl or F.

### Table 105

Table 105 consists of 305,844 compounds. Each of these compounds is exactly the same as the corresponding compound in Tables 1 to 102 (thus, for example, compound 1 of Table 105 is the same as compound 1 of Table 1, compound 663 of Table 105 is the same as compound 1 of Table 2, compound 19,861 of Table 105 is the same as compound 1 of Table 31, compound 305,844 of Table 105 is the same as compound 3,972 of Table 102) except that in all of the compounds of Table 105 R is methyl instead of Cl or F.

### Table 106

Table 106 consists of 305,844 compounds. Each of these compounds is exactly the same as the corresponding compound in Tables 1 to 102 (thus, for example, compound 1 of Table 106 is the same as compound 1 of Table 1, compound 663 of Table 106 is the same as compound 1 of Table 2, compound 19,861 of Table 106 is the same as compound 1 of Table 31, compound 305,844 of Table 106 is the same as compound 3,972 of Table 102) except that in all of the compounds of Table 106 R is H instead of Cl or F.

### Table 107

Table 107 consists of 305,844 compounds. Each of these compounds is exactly the same as the corresponding compound in Tables 1 to 102 (thus, for example, compound 1 of Table 107 is the same as compound 1 of Table 1, compound 663 of Table 107 is the same as compound 1 of Table 2, compound 19,861 of Table 107 is the same as compound 1 of Table 31, compound 305,844 of Table 107 is the same as compound 3,972 of Table 102) except that in all of the compounds of Table 107 R is cyano instead of Cl or F.

### Table 108

Table 108 consists of 305,844 compounds. Each of these compounds is exactly the same as the corresponding compound in Tables 1 to 102 (thus, for example, compound 1 of Table 108 is the same as compound 1 of Table 1, compound 663 of Table 108 is the same as compound 1 of Table 2, compound 19,861 of Table 108 is the same as compound 1 of Table 31, compound 305,844 of Table 108 is the same as compound 3,972 of Table 102) except that in all of the compounds of Table 108 R is methoxy instead of Cl or F.

### Table 109

Table 109 consists of 6620 compounds. Each of these compounds is exactly the same as the corresponding compound in Tables 1 to 10 (thus, for example, compound 1 of Table 109 is the same as compound 1 of Table 1, compound 663 of Table 109 is the same as compound 1 of Table 2, etc.) except that in all of the compounds of Table 109 X is CF instead of CH.

### Table 110

Table 110 consists of 6620 compounds. Each of these compounds is exactly the same as the corresponding compound in Tables 1 to 10 (thus, for example, compound 1 of Table 110 is the same as compound 1 of Table 1, compound 663 of Table 110 is the same as compound 1 of Table 2, etc.) except that in all of the compounds of Table 110 X is CCl instead of CH.

### Table 111

Table 111 consists of 6620 compounds. Each of these compounds is exactly the same as the corresponding compound in Tables 1 to 10 (thus, for example, compound 1 of Table 111 is the same as compound 1 of Table 1, compound 663 of Table 111 is the same as compound 1 of Table 2, etc.) except that in all of the compounds of Table 111 X is CBr instead of CH.

### Table 112

Table 112 consists of 6620 compounds. Each of these compounds is exactly the same as the corresponding compound in Tables 1 to 10 (thus, for example, compound 1 of Table 112 is the same as compound 1 of Table 1, compound 663 of Table 112 is the same as compound 1 of Table 2, etc.) except that in all of the compounds of Table 112 X is CCH₃ instead of CH.

### Table 113

Table 113 consists of 6620 compounds. Each of these compounds is exactly the same as the corresponding compound in Tables 11 to 20 (thus, for example, compound 1 of Table 113 is the same as compound 1 of Table 11, compound 663 of Table 113 is the same as compound 1 of Table 12, etc.) except that in all of the compounds of Table 113 Y is CF instead of CH.

### Table 114

Table 114 consists of 6620 compounds. Each of these compounds is exactly the same as the corresponding compound in Tables 11 to 20 (thus, for example, compound 1 of Table 114 is the same as compound 1 of Table 11, compound 663 of Table 114 is the same as compound 1 of Table 12, etc.) except that in all of the compounds of Table 114 Y is CCl instead of CH.

### Table 115

Table 115 consists of 6620 compounds. Each of these compounds is exactly the same as the corresponding compound in Tables 11 to 20 (thus, for example, compound 1 of Table 115 is the same as compound 1 of Table 11, compound 663 of Table 115 is the same as compound 1 of Table 12, etc.) except that in all of the compounds of Table 115 Y is CBr instead of CH.

### Table 116

Table 116 consists of 6620 compounds. Each of these compounds is exactly the same as the corresponding compound in Tables 11 to 20 (thus, for example, compound 1 of Table 116 is the same as compound 1 of Table 11, compound 663 of Table 116 is the same as compound 1 of Table 12, etc.) except that in all of the compounds of Table 116 Y is CCH₃ instead of CH.

### Table 117

Table 117 consists of 662 compounds of the general formula (1B), where W and Y are N and X and Z are CH, R is F, R¹ is 2,4,6-trifluorophenyl, and the values of R³ and R⁴ are as listed in Table 1. Thus, compound 1 of Table 117 is the same as compound 1 of Table 21 except that in compound 1 of Table 117, the compound has the general formula (1B). Similarly, compounds 2 to 662 of Table 117 are the same as compounds 2 to 662 of Table 21 except that in the compounds of Table 117, the compounds have the general formula (1B).

### Table 118

Table 118 consists of 662 compounds of the general formula (1B where W and Y are N and X and Z are CH, R is F, R¹ is 2,5,6-trifluorophenyl, and the values of R³ and R⁴ are as listed in Table 1. Thus, compound 1 of Table 118 is the same as compound 1 of Table 22 except that in compound 1 of Table 118, the compound has the general formula (1B). Similarly, compounds 2 to 662 of Table 118 are the same as compounds 2 to 662 of Table 22 except that in the compounds of Table 118, the compounds have the general formula (1B).

### Table 119

Table 119 consists of 662 compounds of the general formula (1B), where W and Y are N and X and Z are CH, R is F, R¹ is 2,3,4,5,6-pentafluorophenyl, and the values of R³ and R⁴ are as listed in Table 1. Thus, compound 1 of Table 119 is the same as compound 1 of Table 23 except that in compound 1 of Table 119, the compound has the general formula (1B). Similarly, compounds 2 to 662 of Table 119 are the same as compounds 2 to 662 of Table 23 except that in the compounds of Table 119, the compounds have the general formula (1B).

### Table 120

Table 120 consists of 662 compounds of the general formula (1B), where W and Y are N and X and Z are CH, R is F, R¹ is 2,6-difluoro-4-methoxyphenyl, and the values of R³ and R⁴ are as listed in Table 1. Thus, compound 1 of Table 120 is the same as compound 1 of Table 24 except that in compound 1 of Table 120, the compound has the general formula (1B). Similarly, compounds 2 to 662 of Table 120 are the same as compounds 2 to 662 of Table 24 except that in the compounds of Table 120, the compounds have the general formula (1B).

### Table 121

Table 121 consists of 662 compounds of the general formula (1B), where W and Y are N and X and Z are CH, R is F, R¹ is 2-fluoro-6-chlorophenyl, and the values of R³ and R⁴ are as listed in Table 1. Thus, compound 1 of Table 121 is the same as compound 1 of Table 25 except that in compound 1 of Table 121, the compound has the general formula (1B). Similarly, compounds 2 to 662 of Table 121 are the same as compounds 2 to 662 of Table 25 except that in the compounds of Table 121, the compounds have the general formula (1B).

### Table 122

Table 122 consists of 662 compounds of the general formula (1B), where W and Y are CH and X and Z are N, R is F, R¹ is 2,4,6-trifluorophenyl, and the values of R³ and R⁴ are as listed in Table 1. Thus, compound 1 of Table 122 is the same as compound 1 of Table 26 except that in compound 1 of Table 122, the compound has the general formula (1B). Similarly, compounds 2 to 662 of Table 122 are the same as compounds 2 to 662 of Table 26 except that in the compounds of Table 122, the compounds have the general formula (1B).

### Table 123

Table 123 consists of 662 compounds of the general formula (1B), where W and Y are CH and X and Z are N, R is F, R¹ is 2,4,5-trifluorophenyl, and the values of R³ and R⁴ are as listed in Table 1. Thus, compound 1 of Table 123 is the same as compound 1 of Table 27 except that in compound 1 of Table 123, the compound has the general formula (1B). Similarly, compounds 2 to 662 of Table 123 are the same as compounds 2 to 662 of Table 27 except that in the compounds of Table 123, the compounds have the general formula (1B).

### Table 124

Table 124 consists of 662 compounds of the general formula (1B), where W and Y are CH and X and Z are N, R is F, R¹ is 2,3,4,5,6-pentafluorophenyl, and the values of R³ and R⁴ are as listed in Table 1. Thus, compound 1 of Table 124 is the same as compound 1 of Table 28 except that in compound 1 of Table 124, the compound has the general formula (1B). Similarly, compounds 2 to 662 of Table 124 are the same as compounds 2 to 662 of Table 28 except that in the compounds of Table 124, the compounds have the general formula (1B).

### Table 125

Table 125 consists of 662 compounds of the general formula (1B), where W and Y are CH and X and Z are N, R is F, R¹ is 2,6-difluoro-4-methoxyphenyl, and the values of R³ and R⁴ are as listed in Table 1. Thus, compound 1 of Table 125 is the same as compound 1 of Table 29 except that in compound 1 of Table 125, the compound has the general formula (1B). Similarly, compounds 2 to 662 of Table 125 are the same as compounds 2 to 662 of Table 29 except that in the compounds of Table 125, the compounds have the general formula (1B).

### Table 126

Table 126 consists of 662 compounds of the general formula (1B), where W and Y are CH and X and Z are N, R is F, R¹ is 2-fluoro-6-chlorophenyl, and the values of R³ and R⁴ are as listed in Table 1. Thus, compound 1 of Table 126 is the same as compound 1 of Table 30 except that in compound 1 of Table 126, the compound has the general formula (1B). Similarly, compounds 2 to 662 of Table 126 are the same as compounds 2 to 662 of Table 30 except that in the compounds of Table 126, the compounds have the general formula (1B).

Compounds of formula (7) or (8), which are examples of compounds of general formula (1) where one ofR and R² is NR³R⁴, can be made as shown in Scheme 1, in which W, X, Y, Z, R¹, R³ and R⁴ have the meanings given above and R⁷ is C₁₋₄ alkyl.

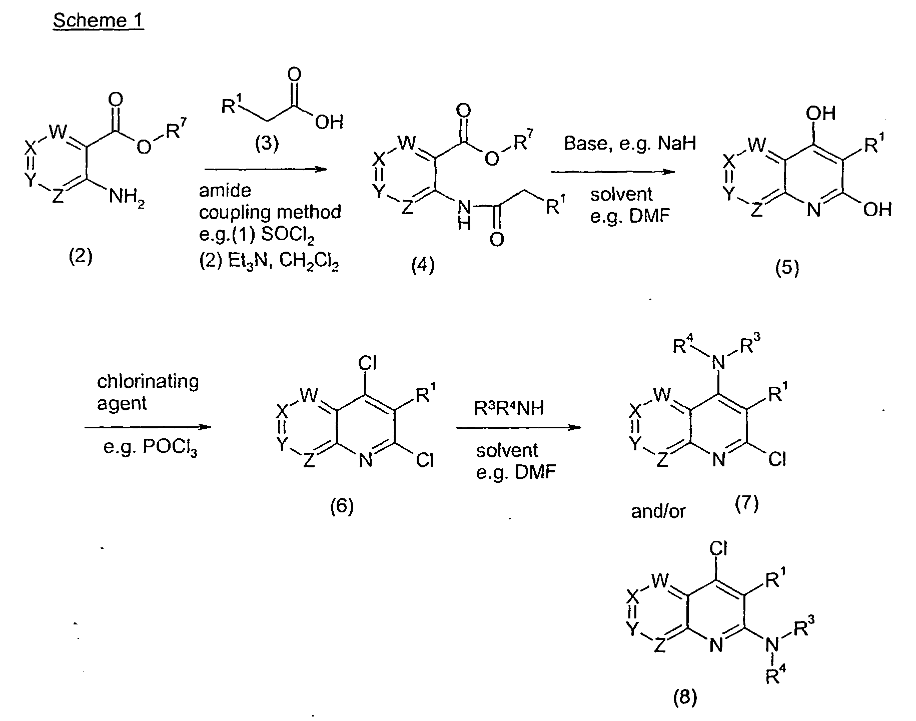

Compounds of general formula (4) can be prepared from compounds of general formula (2), which are either commercially available or made by methods known in the literature, by reaction with acids of general formula (3), using standard coupling methods, for example by conversion to the acid chloride using a chlorinating agent such as thionyl chloride, followed by reaction of the resultant acid chloride optionally in the presence of a base such as triethylamine, in a suitable solvent such as dichloromethane or toluene. Compounds of general formula (5) can be prepared by treating compounds of general formula (4) with a base such as sodium hydride, optionally in the presence of a Lewis acid such as magnesium oxide, in a suitable solvent such as *N,N-*dimethylformamide (DMF) or toluene, at between room temperature and 150°C, but preferably at 60-90°C. Compounds of general formula (6) can be prepared by reaction of compounds of general formula (5) with a chlorination reagent such as phosphorus oxychloride, either neat or in a suitable solvent such as toluene, at between 50 and 150°C, but preferably between 80 and 110°C, or in a microwave reactor at between 150 and 300°C, but preferably between 200 and 250°C. Compounds of formula (7) and (8) can be prepared by reaction of compounds of general formula (6) with an amine R³R⁴NH, either neat, or in a suitable solvent such as DMF, between room temperature and 150°C, but preferably between 50 and 80°C. If compounds (7) and (8) are produced as a mixture they can be separated by suitable means such as crystallisation or chromatography under normal or reverse phase conditions.

Compounds of the general formulae (5), (6), (7) and (8) may be derivatised, via the chloro or hydroxy substituents, using routine chemical techniques to form other compounds of the general formula (1). Alternatively, other compounds of the general formula (1) may be prepared using a similar methodology to that described for preparing the compounds (5) to (8) and employing preparative techniques known from the chemical literature.

Compounds of formula (7) can also be made as shown in Scheme 2.

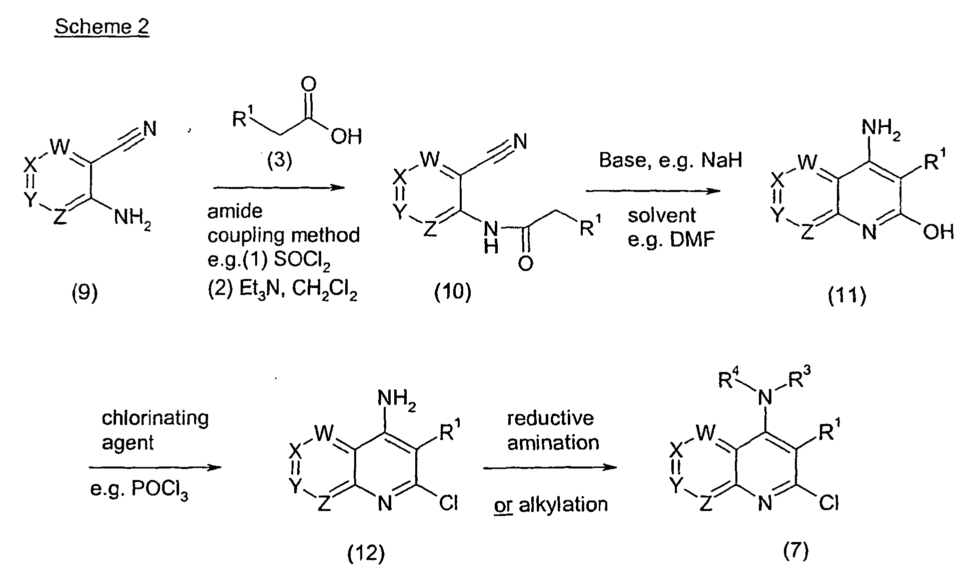

Compounds of general formula (10) can be prepared from compounds of general formula (9), which are either commercially available or made by methods known in the literature, by reaction with acids of general formula (3), using standard coupling methods, for example by conversion to the acid chloride using a chlorinating agent such as thionyl chloride, followed by reaction of the resultant acid chloride optionally in the presence of a base such as triethylamine, in a suitable solvent such as dichloromethane or toluene. Compounds of general formula (11) can be prepared by treating compounds of general formula (10) with a base such as sodium hydride, optionally in the presence of a Lewis acid such as magnesium oxide, in a suitable solvent such as *N,N*-dimethylformamide (DMF) or toluene, at between room temperature and 150°C, but preferably at 60-90°C. Compounds of general formula (12) can be prepared by reaction of compounds of general formula (11) with a chlorination reagent such as phosphorus oxychloride, either neat or in a suitable solvent such as toluene, at between 50 and 150°C, but preferably between 80 and 110°C, or in a microwave reactor at between 150 and 300°C, but preferably between 200 and 250°C. Compounds of formula (7) can be prepared from compounds of formula (12) by reductive amination, for example by reaction with a ketone or aldehyde in a suitable solvent such as ethanol or toluene, at between room temperature and reflux, optionally in the presence of an acid catalyst such as para-toluenesulphonic acid or a drying agent such as molecular sieves, followed by treatment with a suitable reducing agent such as sodium borohydride, at between -20°C and 40°C, but preferably at room temperature. The aldehyde or ketone is chosen so that the desired groups R³ and R⁴ are formed after reduction of the product of reaction with the amine (12). For example if compounds of formula (12) are reacted with one equivalent of propionaldehyde and then sodium borohydride, compounds of formula (7) where R³ is *n-*propyl, and R⁴ is hydrogen are formed. If required, the reaction can be repeated with a different aldehyde or ketone. For example, if acetone is used for the second reaction, then compounds of formula (7) where R³ is *n*-propyl and R⁴ is *iso*-propyl, are formed. Alternatively compounds of formula (7) can be formed from compounds of formula (12) by alkylation with a group R³LG, where LG is a leaving group, by treatment with a suitable base such as sodium hydride in a solvent such as DMF, or a base such as potassium carbonate in a solvent such as acetone or DMF, at between -78°C and 100°C, but preferably between room temperature and 60°C, followed by treatment with R⁴LG in a second step under the same conditions if required.

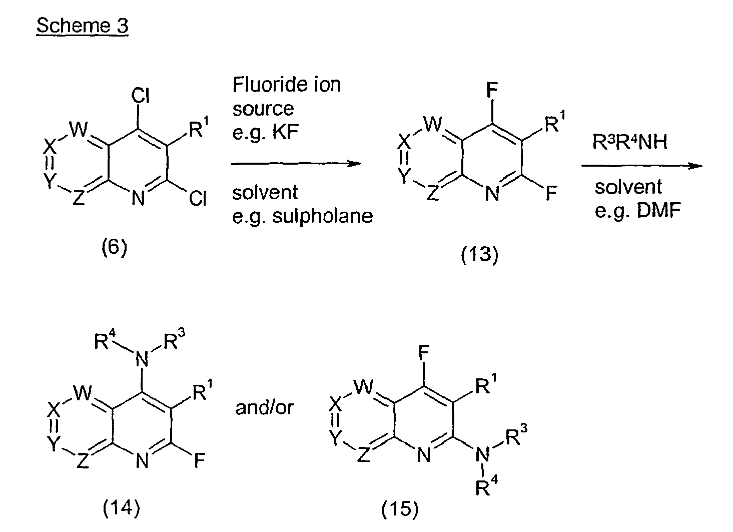

Compounds of formula (13) can be prepared as shown in Scheme 3 from compounds of formula (6) by reaction with a source of fluoride ion, such as potassium fluoride, in a suitable solvent such as sulpholane, at a temperature between 50°C and 200°C, but preferably at 80-150°C. Compounds of formula (14) and/or compounds of formula (15) can be prepared from difluoro compounds of formula (13) by reaction with an amine of formula R³R⁴NH in a suitable solvent such as DMF or CH₂Cl₂, at a temperature of 0°C-100°C, but preferably at room temperature.

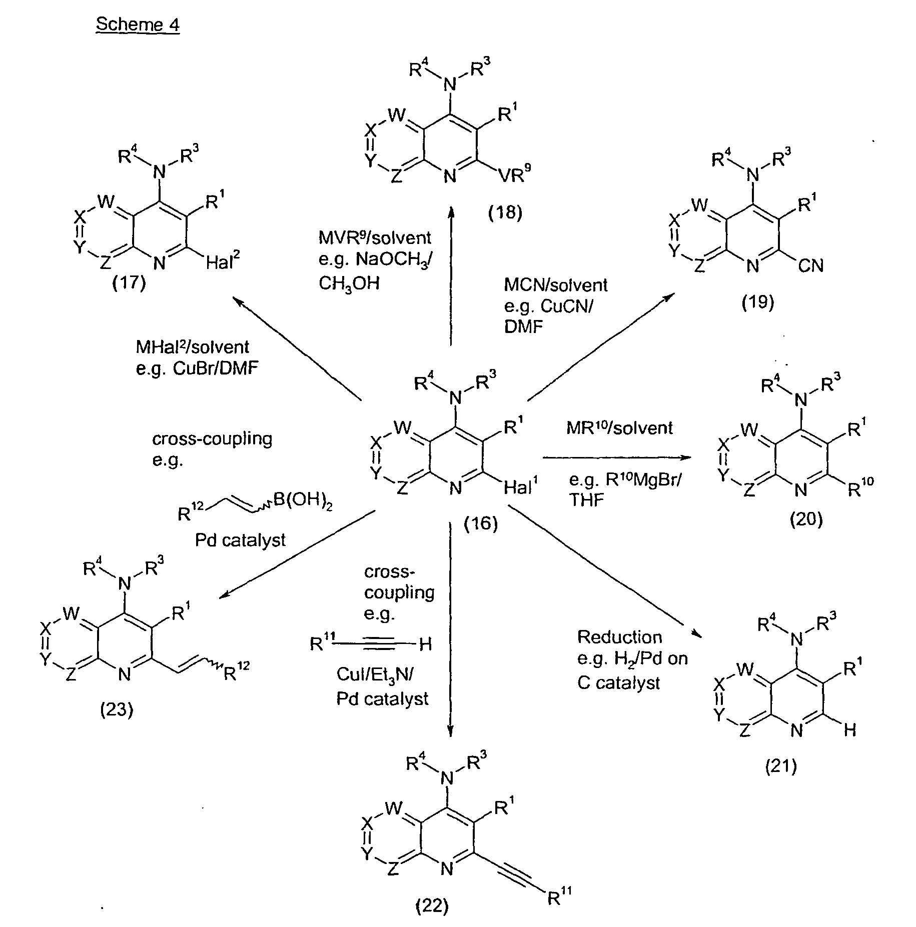

Compounds of general formula (16), where Hall is chlorine or fluorine, can be converted into compounds of formula (17), (18), (19), (20), (21), (22) or (23) as shown in Scheme 4. Compounds of general formula (17) where Hal² is bromine or iodine can be formed by reacting compounds of general formula (16) with a metal halide, for example cuprous bromide, in a suitable solvent, for example DMF, at between room temperature and 155°C, but preferably between 70°C and 155°C. Compounds of general formula (18) where V is oxygen or sulphur and R⁹ is C₁₋₈ alkyl, can be formed by reacting compounds of general formula (16) with a metal alkoxide or thioalkoxide MVR⁹ in a suitable solvent, for example sodium methoxide in methanol, at room temperature to 65°C. Compounds of general formula (19) can be formed by reacting compounds of general formula (16) with a metal cyanide in a suitable solvent, for example cuprous cyanide in DMF, at between room temperature and 155°C but preferably between 50°C and 155°C. Compounds of general formula (20) where R¹⁰ is C₁₋₈ alkyl, can be formed by reacting compounds of general formula (16) with an alkyl metal derivative in a suitable solvent, for example methyl magnesium bromide in THF, optionally in the presence of catalyst such as cuprous bromide or Pd(Ph)₄, between -40°C and 50°C. Compounds of general formula (21) can be formed by reduction of compounds of general formula (16), where Hall is chlorine, for example by hydrogenolysis with hydrogen gas and a metal catalyst such as palladium on carbon in a suitable solvent such as ethanol, at room temperature. Compounds of general formula (22) where R¹¹ is hydrogen or C₁₋₆ alkyl, can be formed by reaction of compounds of general formula (16) with an alkyl acetylene under the Sonogashira conditions, for example with 1-propyne in triethylamine in the presence of a cuprous salt such as cuprous iodide and a palladium catalyst such as Pd(Ph)₄, between room temperature and 70°C. Compounds of general formula (23) where R¹² is hydrogen or C₁₋₆ alkyl, can be formed by reaction of compounds of general formula (16) with an alkenyl metal derivative in a suitable solvent, such as ethenylboronic acid in THF, in the presence of a palladium catalyst such as Pd(Ph)₄ and a base such as caesium carbonate, between room temperature and 65°C.

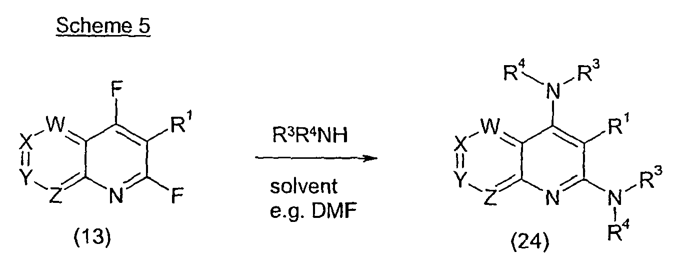

In Scheme 5 compounds of general formula (24), where the two R³R⁴N groups are identical, can be made from compounds of general formula (13) by reaction with a large excess of amine R³R⁴NH in a suitable solvent such as DMF, at a temperature between 0°C and 150°C, but preferably between room temperature and 100°C

Further assistance in the preparation of the compounds of formula (1) may be derived from the following publications: Emilio, Toja, et. al., J. Heterocyclic Chem., 23, 1955 (1986), H. Schäfer, et. al., J. f. prakt. Chemie, 321(4), 695 (1970) and H. Bredereck et. al., Chem. Ber. 96, 1868-1872 (1993).

The intermediate chemicals having the general formulae (4), (5), (6) and (13): 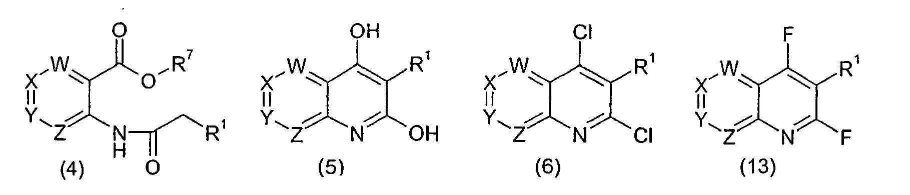 wherein W, X, Y, Z and R¹ are as defined in claim 1 and R⁷ is C₁₋₄ alkyl, other than those compounds of the general formula (5) wherein W and Y are both CH and X and Z are both N and R¹ is methyl, ethyl or phenyl, and other than those compounds of the general formula (5) wherein W is CH, Y is CH₃-C and X and Z are both N and R¹ is methyl, ethyl or phenyl, and other than the compound of the general formula (4) wherein W and Y are both CH₃-C and X and Z are both N and R¹ is methyl and R⁷ is ethyl are believed to be novel compounds and form a further part of this invention.

It should be noted that the intermediate of general formula (5) may exist in the tautomeric forms (a), (b) and (c) as well as in the form shown in formula (5):

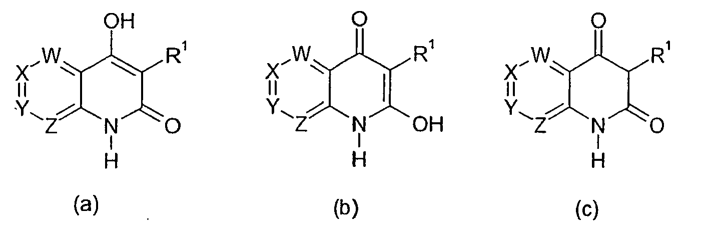

The invention as defined by the general formula (5) embraces all such tautomers.

Of particular interest are the intermediates listed in Tables 127 to 134 below. In Table 127 the compounds have the general formula (4) where R⁷ is methyl and W, X, Y, Z and R¹ have the values shown in the table.

**Table 127**

| Cmpd No. | R¹ | W | X | Y | Z |
|---|---|---|---|---|---|
| 1 | 2,4,6-trifluorophenyl | N | CH | N | CH |
| 2 | 2,5,6-trifluorophenyl | N | CH | N | CH |
| 3 | 2,3,4,5,6-pentafluorophenyl | N | CH | N | CH |
| 4 | 2,3,5,6-tetrafluorophenyl | N | CH | N | CH |
| 5 | 2,6-difluoro-4-methoxyphenyl | N | CH | N | CH |
| 6 | 2-fluoro-6-chlorophenyl | N | CH | N | CH |
| 7 | 2,6-difluorophenyl | N | CH | N | CH |
| 8 | 2,3,5,6-tetrafluorophenyl | N | CH | N | CH |
| 9 | 2-fluorophenyl | N | CH | N | CH |
| 10 | 2-chlorophenyl | N | CH | N | CH |
| 11 | 2-bromophenyl | N | CH | N | CH |
| 12 | 2,4-dichlorophenyl | N | CH | N | CH |
| 13 | 2,4,6-trifluorophenyl | CH | N | CH | N |
| 14 | 2,5,6-trifluorophenyl | CH | N | CH | N |
| 15 | 2,3,4,5,6-pentafluorophenyl | CH | N | CH | N |
| 16 | 2,3,5,6-tetrafluorophenyl | CH | N | CH | N |
| 17 | 2,6-difluoro-4-methoxyphenyl | CH | N | CH | N |
| 18 | 2-fluoro-6-chlorophenyl | CH | N | CH | N |
| 19 | 2,6-difluorophenyl | CH | N | CH | N |
| 20 | 2,3,5,6-tetrafluorophenyl | CH | N | CH | N |
| 21 | 2-fluorophenyl | CH | N | CH | N |
| 22 | 2-chlorophenyl | CH | N | CH | N |
| 23 | 2-bromophenyl | CH | N | CH | N |
| 24 | 2,4-dichlorophenyl | CH | N | CH | N |

### Table 128

Table 128 consists of 24 compounds of the general formula (5), where W, X, Y, Z and R¹ have the values given in Table 127. Thus, compound 1 of Table 128 has the same W, X, Y, Z and R¹ values as compound 1 of Table 127, etc.

### Table 129

Table 129 consists of 24 compounds of the general formula (6), where W, X, Y, Z and R¹ have the values given in Table 127. Thus, compound 1 of Table 129 has the same W, X, Y, Z and R¹ values as compound 1 of Table 127, etc.

### Table 130

Table 130 consists of 24 compounds of the general formula (13), where W, X, Y, Z and R¹ have the values given in Table 127. Thus, compound 1 of Table 130 has the same W, X, Y, Z and R¹ values as compound 1 of Table 127, etc.

### Table 131

Table 131 consists of 24 compounds of the general formula (4), where W, X, Y, Z and R¹ have the values given in Table 127 and R⁷ is ethyl. Thus, compound 1 of Table 131 is the same as compound 1 of Table 127 except that in compound 1 of Table 131, R⁷ is ethyl instead of methyl. Similarly, compounds 2 to 24 of Table 131 are the same as compounds 2 to 24 of Table 127 except that in the compounds of Table 131, R⁷ is ethyl.

The compounds of formula (1) are active fungicides and may be used to control one or more of the following pathogens: *Pyricularia oryzae* (*Magnaporthe grisea*) on rice and wheat and other *Pyricularia* spp. on other hosts; *Puccinia triticina* (or *recondita*), *Pacccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca (Sphaerotheca fuliginea)* on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. (*Pyrenophora* spp.), *Rhynchosporium* spp., *Mycosphaerella graminicola* (*Septaria tritici*) and *Phaeosphaeria nodorum* (*Stagonospora nodorum* or *Septoria nodorum*), *Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (*Glomerella cingulata*), black rot or frogeye leaf spot (*Botryosphaeria obtusa*), Brooks fruit spot (*Mycosphaerella pomi*), Cedar apple rust (*Gymnosporangium juniperi-virginianae*), sooty blotch (*Gloeodes pomigena*), flyspeck (*Schizothyrium pomi*) and white rot (*Botryosphaeria dothidea*)) on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum*) on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramu*/*aria* spp. on sugar beet, barley and other hosts; post-harvest diseases particularly of fruit (for example *Penicillium digitatum*, *Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum;* other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans*; and fungal vectors of viral diseases (for example *Polymyxa graminis* on cereals as the vector of barley yellow mosaic virus (BYMV) and *Polymyxa betae* on sugar beet as the vector of rhizomania).

A compound of formula (1) may move acropetally, basipetally or locally in plant tissue to be active against one or more fungi. Moreover, a compound of formula (1) maybe volatile enough to be active in the vapour phase against one or more fungi on the plant.

The invention therefore provides a method of combating or controlling phytopathogenic fungi which comprises applying a fungicidally effective amount of a compound of formula (1), or a composition containing a compound of formula (1), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The compounds of formula (1) are preferably used for agricultural, horticultural and turfgrass purposes in the form of a composition.

In order to apply a compound of formula (1) to a plant, to a seed of a plant, to the locus of the plant or seed or to soil or any other growth medium, a compound of formula (1) is usually formulated into a composition which includes, in addition to the compound of formula (1), a suitable inert diluent or carrier and, optionally, a surface active agent (SFA). SFAs are chemicals that are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (1). The composition is generally used for the control of fungi such that a compound of formula (1) is applied at a rate of from 0.1g to 10kg per hectare, preferably from 1g to 6kg per hectare, more preferably from 1g to 1kg per hectare.

When used in a seed dressing, a compound of formula (1) is used at a rate of 0.0001g to 10g (for example 0.001g or 0.05g), preferably 0.005g to 10g, more preferably 0.005g to 4g, per kilogram of seed.

In another aspect the present invention provides a fungicidal composition comprising a fungicidally effective amount of a compound of formula (1) and a suitable carrier or diluent therefor.

In a still further aspect the invention provides a method of combating and controlling fungi at a locus, which comprises treating the fungi, or the locus of the fungi with a fungicidally effective amount of a composition comprising a compound of formula (1).

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (1).

Dustable powders (DP) may be prepared by mixing a compound of formula (1) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (1) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (1) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (1) and one or more powdered solid diluents or carriers, or from pre-formed blank granules by absorbing a compound of formula (1) (or a solution thereof, in a suitable agent) in a porous granular material (such as pumice, attapulgite clays, fuller's earth, kieselguhr, diatomaceous earths or ground corn cobs) or by adsorbing a compound of formula (1) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (1) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (1) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), N-alkylpyrrolidones (such as *N*-methylpyrrolidone or *N*-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (1) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents that have a low solubility in water.

Microemulsions (ME) may be prepared by mixing water with a blend of one or more solvents with one or more SFAs, to produce spontaneously a thermodynamically stable isotropic liquid formulation. A compound of formula (1) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (1). SCs may be prepared by ball or bead milling the solid compound of formula (1) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (1) may be dry milled and added to water, containing agents hereinbefore described, to produce the desired end product.

Aerosol formulations comprise a compound of formula (1) and a suitable propellant (for example *n*-butane). A compound of formula (1) may also be dissolved or dispersed in a suitable medium (for example water or a water miscible liquid, such as *n*-propanol) to provide compositions for use in non-pressurised, hand-actuated spray pumps.

A compound of formula (1) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (1) and, optionally, a carrier or diluent therefor. The polymeric shell may be produced by either an interfacial polycondensation reaction or by a coacervation procedure. The compositions may provide for controlled release of the compound of formula (1) and they may be used for seed treatment. A compound of formula (1) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (1)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (1)).

A compound of formula (1) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (1) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (1) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (1) (for example 0.0001 to 10%, by weight) depending upon the purpose for which they are to be used.

A compound of formula (1) may be used in mixtures with fertilisers (for example nitrogen-, potassium- or phosphorus-containing fertilisers). Suitable formulation types include granules of fertiliser. The mixtures suitably contain up to 25% by weight of the compound of formula (1).

The invention therefore also provides a fertiliser composition comprising a fertiliser and a compound of formula (1).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (1) alone. Further the other fungicide may have a synergistic effect on the fungicidal activity of the compound of formula (1).

The compound of formula (1) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (1); or help to overcome or prevent the development of resistance to individual components. The particular additional active ingredient will depend upon the intended utility of the composition.

Examples of fungicidal compounds which may be included in the composition of the invention are AC 382042 (*N*-(1-cyano-1,2-dimethylpropyl)-2-(2,4-dichlorophenoxy) propionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan, carbendazim, carbendazim chlorhydrate, carboxin, carpropamid, carvone, CGA 41396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, *O,O*-di-*iso*-propyl-*S*-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (*Z*)-*N*-benzyl-*N*([methyl(methylthioethylideneaminooxycarbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (N -allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyanomethylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin, XRD-563, zineb, ziram, zoxamide and compounds of the formulae:

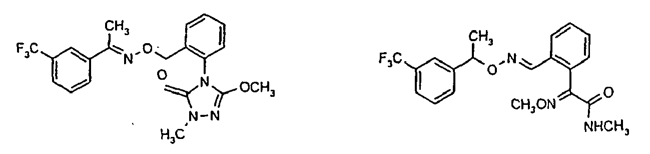

The compounds of formula (1) may be mixed with soil, peat or other rooting media for the protection of plants against seed-borne, soil-bome or foliar fungal diseases.

Some mixtures may comprise active ingredients, which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples in which the following abbreviations are used:

| | |
|---|---|
| ml = millilitres | m.p. = melting point |
| g = grammes | THF = tetrahydrofuran |
| ppm = parts per million | DCM = dichloromethane |
| s = singlet | DMF = N, *N*-dimethylformamide |
| d = doublet | DMSO = dimethylsulphoxide |
| t = triplet | DMAP = 4-dimethylaminopyridine |
| q = quartet | NMR = nuclear magnetic resonance |
| m = multiplet | EDC = 1-ethyl-3-*N,N*-dimethylamino-propylcarbodiimide hydrochloride |
| b = broad | |

### EXAMPLE 1

This Example illustrates the preparation of [6-chloro-7-(2,4,6-trifluorophenyl)-pyrido[3,2-d]pyrimidin-8-yl]-isopropylamine, Compound No. 3 from Table 1.

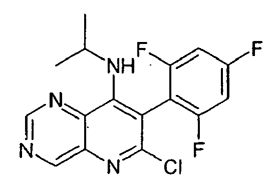

### Step1

Potassium 5-nitro-2,6-dioxo-1,2,3,6-tetrahydro-pyrimidine-4-carboxylate (40.0 g) was refluxed in ethanol (400 ml) containing concentrated sulphuric acid (60 ml) for 8 hours. The mixture was cooled and filtered and the solid washed with ethanol to give 5-nitro-2,6-dioxo-1,2,3,6-tetrahydro-pyrimidine-4-carboxylic acid ethyl ester as a white powder (49.54 g), m.p. 259°C
¹H NMR (d⁶-DMSO) δ ppm: 1.28 (t,3H), 4.35 (q,2H), 5.10 (bs, 1H), 12.0 (s,1 H)

### Step 2

Phosphorus oxychloride (156 ml) was added to a mixture of the product from Step 1 (15.4 g) and *N,N*-diethylaniline (20 ml) and stirred for 30 minutes at room temperature. The reaction was then refluxed for 1.5 hour and the phosphorus oxychloride was evaporated, and the residue was put on ice and extracted with diethyl ether. The ether fraction was washed with 2 M hydrochloric acid, saturated sodium bicarbonate and water, and then the ether was evaporated. The residue was purified by column chromatography on silica gel (40-60) eluting with hexane:ethyl acetate (4:1) to give 2,4-dichloro-6-nitro-pyrimidine-5-carboxylic acid methyl ester as a light brown oil (15.5 g).
¹H NMR (d⁶-DMSO) δ ppm: 1.4 (t,3H), 4.5 (q,2H)

### Step 3

The product from step 2 (2.64 g) was hydrogenated at room temperature at a pressure of 3.75 bar hydrogen gas for 5 hours, in dioxane (180 ml) and in the presence of magnesium oxide (1.96 g) and 5% palladium on carbon (2.44 g). The reaction mixture was filtered through celite, washed with dioxane, ethyl acetate, and methanol. The solvents were combined and evaporated and the reaction mixture was purified by column chromatography on silica gel (40-60) eluting with ethyl acetate to give 5-amino-2-chloro-pyrimidine-4-carboxylic acid ethyl ester as a yellow solid (1.38 g).
¹H NMR (CDCl₃) δ ppm: 1.45 (t,3H), 4.5 (q,2H), 5.75 (bs,2H), 8.3 (s, 1H).

### Step 4

The product from Step 3 (0.64 g) in methanol (60 ml) and triethylamine (0.66 ml) was hydrogenated at a pressure of 3.50 bar hydrogen gas in the presence of 5% palladium on carbon (0.64 g) for 3 hours. The reaction mixture was filtered through celite and washed with methanol, and the methanol was evaporated. The residue was purified by column chromatography on silica gel (40-60) eluting with hexane:ethyl acetate (1:2) to give 5-aminopyrimidine-4-carboxylic acid ethyl ester as a yellow solid (0.150 g).
¹H NMR (CDCl₃) δ ppm: 1.48 (t,3H), 4.48 (q,2H), 5.71 (bs, 2 H), 8.41 (s, 1H), 8.68 (s, 1H)

### Step 5

To the product from Step 4 (0.755 g) in dry DCM (50 ml) in an ice bath was added pyridine (0.73 ml), followed after 5 minutes by a slow addition of 2,4,6-trifluorophenylacetyl chloride (1.41 g) in dry DCM (10 ml). The reaction mixture was stirred for 3 hours and allowed to warm to room temperature. A further portion of 2,4,6-trifluorophenylacetyl chloride (0.3 g) was added and the reaction was stirred at room temperature for 1 hour. The DCM was evaporated, and the residue was purified by column chromatography on silica gel (40-60) eluting with hexane:ethyl acetate (1:1), to give 5-[2-(2,4,6-trifluorophenyl)-acetylamino]-pyrimidine-4-carboxylic acid ethyl ester as a white-solid (1.1 g).
¹H NMR (CDCl₃) δ ppm: 1.48 (t,3H), 3.88 (s,2H), 4.5 (q,2H), 6.78 (m,2H), 9.06 (s, 1H), 10.2 (s, 1H), 10.8 (s, 1H).

### Step 6

The product from Step 5 (0.55 g) was heated with potassium carbonate (0.056 g) in dry DMF (25 ml) at 125°C for 1 hour. The DMF was evaporated, and the residue treated with water and acidified with dilute aqueous hydrochloric acid, and then extracted with ethyl acetate. The ethyl acetate was dried over magnesium sulphate, filtered and evaporated. The residue was purified by column chromatography on silica gel (40-60) eluting with ethyl acetate:methanol (1:1), and then triturated with diethyl ether to give 6-hydroxy-7-(2,4,6-trifluorophenyl)-5*H*-pyrido[3,2-d]pyrimidin-8-one as a brown solid (0.083 g).
¹H NMR (d⁶-DMSO) δ ppm: 7.12 (m,2H), 8.71 (s, 1H), 8.87 (bs, 1H), 10.82 (bs, 1H).

### Step 7

The product of Step 6 (0.015 g) was heated with phenyl phosphonic dichloride (1.5 ml) at 165°C overnight, then cooled and poured onto ice, and extracted with ethyl acetate. The organic fraction was dried over magnesium sulphate, and evaporated. The residue was purified by column chromatography on silica gel (40-60) eluting with hexane:ethyl acetate (1:1), to give 6,8-dichloro-7-(2,4,6-trifluorophenyl)-pyrido[3,2-d]pyrimidine (0.007 g).
¹H NMR (CDCl₃) δ ppm: 6.9 (m,2H), 9.58 (s, 1H), 9.68 (s, 1H).

### Step 8

The product from Step 7 (0.007 g) was stirred in isopropylamine (2 ml) at room temperature overnight in sealed tube. The reaction mixture was evaporated and the residue was purified by column chromatography on silica gel (40-60) eluting with hexane:ethyl acetate (2:1) to give the title compound [6-chloro-7-(2,4,6-trifluorophenyl)-pyrido[3,2-d]pyrimidin-8-yl]-isopropylamine (0.004 g).
¹H NMR (CDCl₃) δ ppm: 1.12 (d,6H), 3.32 (m, 1H), 6.82 (t,2H), 6.92 (m, 1H), 9.2 (s, 1H), 9.38 (s, 1H).

### EXAMPLE 2

This Example illustrates the preparation of sec-butyl-[7-chloro-6-(2,4,6-trifluorophenyl)-pyrido[2,3-d]pyrimidin-5-yl]-amine (Compound No. 23 from Table 11) and *sec*-butyl-[5-chloro-6-(2,4,6-trifluorophenyl)-pyrido[2,3-d]pyrimidin-7-yl]-amine (Compound No. 23 from Table 16)

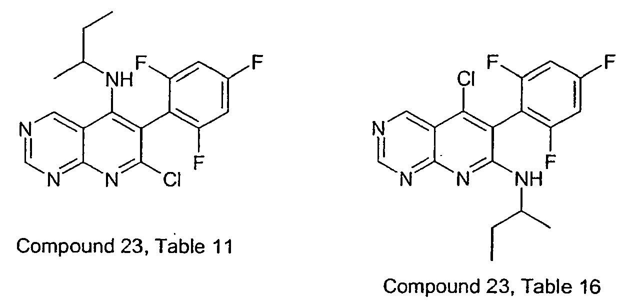

### Step 1

4-Amino-pyrimidine-5-carboxylic acid hydrochloride (10.4 g) was suspended in ethanol (300 ml) at room temperature. DMAP (14.6 g) was added in one portion and the reaction stirred to give a clear yellow solution. EDC (11.5 g) was then added, and the reaction was stirred at room temperature for 17 hours. The solvent was removed to leave a solid, which was dissolved in water and extracted with DCM (2x 200ml). The DCM fraction was dried over magnesium sulphate, and then evaporated to give 4-amino-pyrimidine-5-carboxylic acid methyl ester as a buff solid (16.2 g), as a 1:1 mixture with DMAP, which was used without further purification.
¹H NMR (CDCl₃) δ ppm: 1.35 (t,3H), 4.35 (q,2H), 6.7 (bs, 1H), 7.8 (bs, 1H), 8.5 (s, 1H), 8.9 (s, 1H).

### Step 2

The crude product from Step 1 (13.5 g) was dissolved in dry DCM (100ml) and then pyridine (10ml) was added giving a dark brown solution. The stirred solution was cooled in an ice bath, and the 2,4,6-trifluorophenylacetyl chloride (9.0 g) in dry DCM (100ml) was added dropwise. The mixture was stirred for 6 hours and stood overnight. A further portion of acid chloride (3.0 g) was added and the reaction was stirred for another 10 hours. The solvent was evaporated to give an orange sludge. Water was added and the solid was dissolved in ethyl acetate and then washed with sodium bicarbonate solution and a small amount of 1M hydrochloric acid (10 ml) solution. The water and the ethyl acetate fraction was dried over magnesium sulphate. The solvent was evaporated to yield a solid, which was triturated with ether to give 4-[2-(2,4,6-trifluorophenyl)-acetylamino]-pyrimidine-5-carboxylic acid ethyl ester as a buff coloured solid (10.3 g).
¹H NMR (CDCl₃) δ ppm: 1.45 (t,3H), 4.2 (s,2H), 4.45 (q,2H) 6.75 (t,2H), 9.05 (bs, 1H), 9.2 (s, 1H), 11.0 (s, 1H).

### Step 3

The product from Step 2 (0.90 g) was dissolved in DMF (10 ml) and was added dropwise to a stirred suspension of sodium hydride (0.32 g of a 60% dispersion in oil) in DMF (20 ml). The reaction was stirred at room temperature for 2 hours and then heated to 80°C for 8 hours. The reaction mixture was cooled and the DMF was evaporated to give a yellow solid, which was then acidified with dilute aqueous hydrochloric acid. The white suspension was filtered and collected, washed with diethyl ether and dried to give 6-(2,4,6-trifluorophenyl)-8H-pyrido[2,3-d]pyrimidine-5,7-dione (0.30 g).
¹H NMR (CD₃OD) δ ppm: 7.0 (t,2H), 9.0 (s, 1H), 9.25 (s, 1H).

### Step 4

The product from Step 3 (0.12 g) was suspended in phosphorus oxychloride (5.0 ml) and stirred at room temperature. Phosphorus pentachloride (0.10 g) was added and the reaction was brought to reflux for 17 hours. The reaction was cooled and solvents were evaporated to give a brown oil, which was poured onto ice and stirred for 10 minutes. The crude mixture was extracted with ethyl acetate, and the organic fraction was washed with saturated sodium bicarbonate and then dried over magnesium sulphate. The solvent was evaporated to give 5,7-dichloro-6-(2,4,6-trifluorophenyl)-pyrido[2,3-d]pyrimidine as a brown solid (0.075 g).
¹H NMR (CDCl₃) δ ppm: 6.5 (bs,H), 6.75 (t,2H), 9.1 (bs, 1H), 11.4 (s, 1H).

### Step 5

The product from Step 4 (0.1 g) was dissolved in DMF (2 ml). *Sec*-butylamine (0.05 ml) and DMAP (0.01 g) were added and the mixture was stirred in a sealed tube at 40°C for 14 hours. The yellow coloured reaction mixture was diluted with diethyl ether, washed with brine and dried over sodium sulphate. After evaporation of the solvent the resulting yellow gum, which w as a mixture of two isomers, was purified by flash column chromatography on silica gel (40-60) in toluene:diethyl ether 1:1 to give two isomers:
*sec*-butyl-[7-chloro-6-(2,4,6-trifluorophenyl)-pyrido[2,3-d]pyrimidin-5-yl]-amine, as a yellow solid (0.04g), m.p. 193 °C:
¹H NMR (CDC13) δ ppm: 0.79 (td,3H), 1.11 (d,3H), 1.49 (m,2H), 4.49 (m, 1H), 4.61 (m, 1H), 6.74 (t,2H), 9.15 (s, 1H), 9.30 (s, 1H);
*sec*-butyl-[5-chloro-6-(2,4,6-trifluorophenyl)-pyrido[2,3-d]pyrimidin-7-yl]-amine as a white solid (0.035g), m.p. 179°C:
¹H NMR (CDCl3) δ ppm: 0.79 (t,3H), 1.15 (d,3H), 1.51 (m,2H), 3.68 (m, 1H), 4.49 (m, 1H), 6.68 (t,2H), 9.32 (s,1H), 9.48 (s, 1H).

### EXAMPLE 3

This Example illustrates the preparation of *sec*-butyl-[7-fluoro-6-(2,4,6-trifluorophenyl)-pyrido[2,3-d]pyrimidin-5-yl]-amine (Compound No. 23 from Table 26).

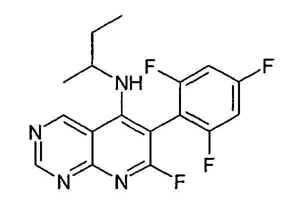

### Step 1

5,7-Dichloro-6-(2,4,6-trifluorophenyl)-pyrido[2,3-d]pyrimidine (0.3 g) was dissolved in dry sulpholane (2 ml). Spray dried potassium fluoride (0.16 g) was added and the mixture was heated in a sealed tube at 100°C for 14 hours. The honey coloured reaction mixture was cooled to ambient temperature, diluted with diethyl ether, and then washed with water to give a yellow oil, which was purified by flash column chromatography on silica gel (40-60) eluting with toluene:diethyl ether, 1:1, to give 5,7-difluoro-6-(2,4,6-trifluorophenyl)-pyrido[2,3-d]pyrimidine as a light yellow solid, m.p. 248 °C
¹H NMR (CDCl3) δ ppm: 6.82 (td,2H), 9.58 (s, 1H), 9.79 (s, 1H).

### Step 2

The product from Step 1 (0.13g) was dissolved in DMF (4 ml). *Sec*-butylamine (0.2 ml) and DMAP (0.01 g) were added and the mixture was stirred in a sealed tube at 40°C for 14 hours. The light yellow coloured reaction mixture was diluted with diethyl ether, washed with brine and dried over sodium sulphate. After evaporation of the solvent the resulting colourless gum was purified by flash column chromatography on silica gel (40-60) in toluene:diethyl ether 1:1 to give sec-butyl-[7-fluoro-6-(2,4,6-trifluorophenyl)-pyrido[2,3-d]pyrimidin-5-yl]-amine as a white solid (0.08g), m.p.199 °C:
¹H NMR (CDCl3) δ ppm: 0.78 (m,3H), 1.21 (t,3H), 1.51 (m,2H), 4.02 (m,2H), 6.71 (t,2H), 9.09 (s, 1H), 9.21 (s, 1H).

**Table 132**

| Compound No. | Table No. | Compound Structure | NMR data (ppm, in CDCl₃, unless otherwise stated) or Mpt. |
|---|---|---|---|
| 3 | 1 | 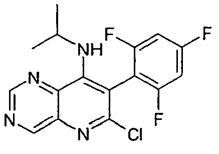 | 1.12 (d,6H), 3.32 (m, 1H), 6.82 (t,2H), 6.92 (m, 1H), 9.2 (s, 1H), 9.38 (s, 1H). |
| 23 | 11 | 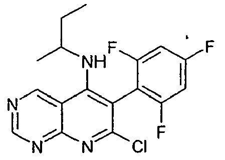 | 191-193°C |
| 23 | 16 | 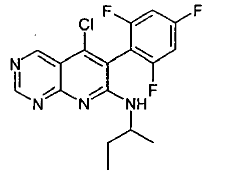 | 176-179°C |
| 23 | 26 | 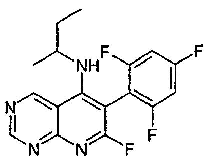 | 199°C |
| 1 | 131 | 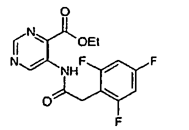 | 1.48 (t,3H), 3.88 (s,2H), 4.5 (q,2H), 6.78 (m,2H), 9.06 (s, 1H), 10.2 (s, 1H), 10.8 (s, 1H). |
| 1 | 128 | 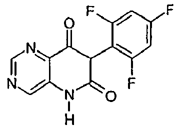 | 7.12 (m,2H), 8.71 (s, 1H), 8.87 (bs, 1H), 10.82 (bs, 1H). |
| 1 | 129 | 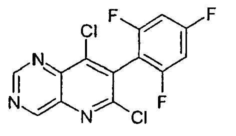 | 6.9 (m, 2H), 9.58 (s, 1H), 9.68 (s, 1H). |
| 13 | 127 | 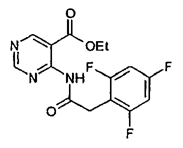 | 1.45 (t,3H), 4.2 (s,2H), 4.45 (q,2H) 6.75 (t,2H), 9.05 (bs, 1H), 9.2 (s, 1H), 11.0 (s, 1H |
| 13 | 128 | 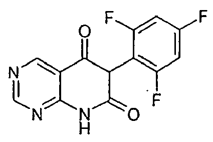 | 7.0 (t,2H), 9.0 (s, 1H),9.25 (s, 1H). |
| 13 | 129 | 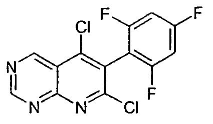 | 6.5 (bs,H), 6.75 (t,2H), 9.1 (bs, 1H), 11.4 (s, 1H). |
| 13 | 130 | 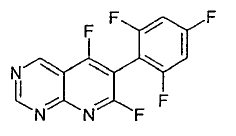 | 248°C |

### EXAMPLE 4

This Example illustrates the fungicidal properties of the compounds of the general formula (1).

Compounds were tested in a leaf disk assay, with methods described below. Test compounds were dissolved in DMSO, and diluted into water to 200 ppm. *Erysiphe graminis f.sp. hordei* (barley powdery mildew): barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity. *Erysiphe graminis f.sp. tritici* (wheat powdery mildew): wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity. *Pyricularia oryzae* (rice blast): rice leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
The following compounds gave greater than 60% control of disease (number of compound first, followed by table number in brackets):
*Erysiphe graminis f.sp. hordei*, Compound 3 (1);
*Erysiphe graminis f.sp. tritici,* Compound 3 (1);
*Pyricularia oryzae*, Compound 3 (1).

## Claims

1. The compound of the general formula (1): 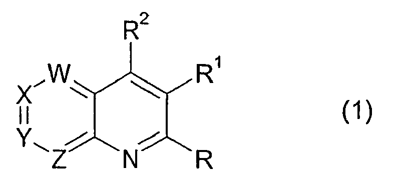 wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
R and R² are independently H, halo, C₁₋₈ alkyl, C₁₋₈ alkoxy, C₁₋₈ alkylthio, C₂₋₈ alkenyl, C₂₋₈ alkynyl, cyano or NR³R⁴, provided that at least one of R and R² is NR³R⁴;
R¹ is halo, C₁₋₈ alkyl, C₂₋₈ alkenyl, C₂₋₈ alkynyl, C₃₋₈ cycloalkyl, C₃₋₈ cycloalkyl(C₁₋₆)-alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl(C₁₄)alkoxy, aryl(C₁₋₄)alkylthio, heteroaryl(C₁₋₄)alkylthio, morpholino, piperidino or pyrrolidino;
R³ and R⁴ are independently H, C₁₋₈ alkyl, C₂₋₈ alkenyl, C₂₋₈ alkynyl, aryl, aryl(C₁₋₈)-alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are H or NR⁵R⁶, or
R³ and R⁴ together form a C₃₋₇ alkylene or C₃₋₇ alkenylene chain optionally substituted with one or more C₁₋₄ alkyl or C₁₋₄ alkoxy groups, or, together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring; and
R⁵ and R⁶ are independently H, C₁₋₈ alkyl, C₂₋₈ alkenyl, C₂₋₈ alkynyl, aryl, aryl(C₁₋₈)-alkyl, C₃₋₈ cycloalkyl, C₃₋₈ cycloalkyl(C₁₋₆)alkyl, heteroaryl or heteroaryl(C₁₋₈)alkyl; any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and any of the foregoing aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy-(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" or -N=CR"'R"", in which R"' and R"" are independently hydrogen, C₁₋₄ alkyl, halo-(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy;
provided that Y is not CCH₃ when W is CH, X and Z are N, R is NHCH₃, R¹ is 2,6-dichlorophenyl and R² is H.

2. A compound according to claim 1 wherein W and Y are both N and X and Z are both CH or X and Z are both N and W and Y are both CH.

3. A compound according to claim 1 or 2 wherein R² is NR³R⁴.

4. A compound according to claim 3 wherein R is halo.

5. A compound according to any one of the preceding claims wherein
R³ is C₁₋₈ alkyl, halo(C₁₋₈)alkyl, hydroxy(C₁₋₈)alkyl, C₁₋₄ alkoxy(C₁₋₈)alkyl, C₁₋₄ alkoxyhalo(C₁₋₈)alkyl, tri(C₁₋₄)alkylsilyl(C₁₋₆)alkyl, C₁₋₄ alkylcarbonyl(C₁₋₈)alkyl, C₁₋₄ alkylcarbonylhalo(C₁₋₈)alkyl, phenyl(₁₋₄)alkyl, C₂₋₈ alkenyl, halo(C₂₋₈)alkenyl, C₂₋₈ alkynyl, C₃₋₈ cycloalkyl optionally substituted with chloro, fluoro or methyl, C₃₋₈ cycloalkyl(C₁₋₄)alkyl, phenylamino, piperidino or morpholino, the phenyl ring of phenylalkyl or phenylamino being optionally substituted with one, two or three substituents selected from halo, C₁₋₄ alkyl, halo(C₁₋₄)alkyl, C₁₋₄ alkoxy and halo-(C₁₋₄)alkoxy; and
R⁴ is H, C₁₋₄ alkyl, halo(C₁₋₄)alkyl or amino, or
R³ and R⁴ together form a C₃₋₇ alkylene or alkenylene chain optionally substituted with methyl, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring, in which the morpholine or piperazine rings are optionally substituted with methyl.

6. A compound according to any one of the preceding claims wherein
R¹ is phenyl optionally substituted with from one to five halogen atoms or with from one to three substituents selected from halo, C₁₋₄ alkyl, halo(C₁₋₄)alkyl, C₁₋₄ alkoxy or halo(C₁₋₄)alkoxy, pyridyl optionally substituted with from one to four halogen atoms or with from one to three substituents selected from halo, C₁₋₄ alkyl, halo(C₁₋₄)alkyl, C₁₋₄ alkoxy or halo(C₁₋₄)alkoxy, 2- or 3-thienyl optionally substituted with from one to three halogen atoms or with from one to three substituents selected from halo, C₁₋₄ alkyl, halo(C₁₋₄)alkyl, C₁₋₄ alkoxy or halo(C₁₋₄)alkoxy, or piperidino or morpholino both optionally substituted with one or two methyl groups.

7. A compound according to claim 6 wherein R¹ is 2,6-difluorophenyl, 2-fluoro-6-chlorophenyl, 2,5,6-trifluorophenyl, 2,4,6-trifluorophenyl, 2,6-difluoro-4-methoxyphenyl or pentafluorophenyl.

8. A compound according to claim 1 wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
one of R and R² (preferably R²) is NR³R⁴ and the other is halo;
R¹ is halo, C₁₋₈ alkyl, C₂₋₈ alkenyl, C₂₋₈ alkynyl, C₃₋₈ cycloalkyl, C₃₋₈ cycloalkyl(C₁₋₆)-alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl-(C₁₋₄)alkoxy, aryl(C₁₋₄)alkylthio, heteroaryl(C₁₋₄)alkylthio, morpholino, piperidino or pyrrolidino;
R³ and R⁴ are independently H, C₁₋₈ alkyl, C₂₋₈ alkenyl, C₂₋₈ alkynyl, aryl, aryl(C₁₋₈)-alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are H or NR⁵R⁶, or
R³ and R⁴ together form a C₃₋₇ alkylene or a C₃₋₇ alkylene chain optionally substituted with one or more C₁₋₄ alkyl or C₁₋₄ alkoxy groups, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring; and R⁵ and R⁶ are independently H, C₁₋₈ alkyl, C₂₋₈ alkenyl, C₂₋₈ alkynyl, aryl, aryl(C₁₋₈)-alkyl, C₃₋₈ cycloalkyl, C₃₋₈ cycloalkyl(C₁₋₆)alkyl, heteroaryl or heteroaryl(C₁₋₈)alkyl; any of the foregoing alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and any of the aryl, heteroaryl, aryloxy or heteroaryl groups being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄ alkoxy-(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" or -N=CR"'R"", in which R"' and R"" are independently hydrogen, C₁₋₄ alkyl, halo-(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.

9. A compound according to claim 1 wherein
W and Y are both N and X and Z are both CR⁸ or X and Z are both N and W and Y are both CR⁸;
R⁸ is H, halo, C₁₋₄ alkyl, C₁₋₄ alkoxy or halo(C₁₋₄)alkyl;
one of R and R² (preferably R²) is NR³R⁴ and the other is halo;
R¹ is halo, C₁₋₈ alkyl, C₂₋₈ alkenyl, C₂₋₈ alkynyl, C₃₋₈ cycloalkyl, C₃₋₈ cycloalkyl(C₁₋₆)-alkyl, C₁₋₈ alkoxy, C₁₋₈ alkylthio, aryl, aryloxy, arylthio, heteroaryl, heteroaryloxy, heteroarylthio, aryl(C₁₋₄)alkyl, aryl(C₁₋₄)alkoxy, heteroaryl(C₁₋₄)alkyl, heteroaryl-(C₁₋₄)alkoxy, aryl(C₁₋₄)alkylthio, heteroaryl(C₁₋₄)alkylthio, morpholino, piperidino or pyrrolidino;
R³ is C₁₋₄ alkyl, halo(C₁₋₄)alkyl, C₂₋₄ alkenyl, C₃₋₆ cycloalkyl, C₃₋₆ cycloalkyl(C₁₋₄)-alkyl or phenylamino in which the phenyl ring is optionally substituted with one, two or three substituents selected from halo, C₁₋₄ alkyl, halo(C₁₋₄)alkyl, C₁₋₄ alkoxy and halo(C₁₋₄)alkoxy; and R⁴ is H, C₁₋₄ alkyl or amino, or
R³ and R⁴ together form a C₄₋₆ alkylene chain optionally substituted with C₁₋₄ alkyl or C₁₋₄ alkoxy, or,
together with the nitrogen atom to which they are attached, R³ and R⁴ form a morpholine, thiomorpholine, thiomorpholine S-oxide or thiomorpholine S-dioxide ring or a piperazine or piperazine *N*-(C₁₋₄)alkyl (especially *N*-methyl) ring; any of the alkyl, alkenyl, alkynyl or cycloalkyl groups or moieties (other than for R⁸) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino,
any of the foregoing morpholine, thiomorpholine, piperidine, piperazine and pyrrolidine rings being optionally substituted with C₁₋₄ alkyl (especially methyl), and any of the aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)-alkoxy, C₁₋₆ alkylthio, halo(C₁₋₆)alkylthio, hydroxy(C₁₋₆)alkyl, C₁₋₄ alkoxy(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R''', -OSO₂R''', -COR''', -CR"'=NR"" or -N=CR"'R"", in which R"' and R"" are independently hydrogen, C₁₋₄ alkyl, halo(C₁₋₄)alkyl, C₁₋₄ alkoxy, halo-(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.

10. A process for preparing a compound of the general formula (1) according to claim 1 wherein one of R and R² is chloro or fluoro and the other is NR³R⁴ and W, X, Y, Z, R¹, R³ and R⁴ are as defined in claim 1, which comprises reacting an amine of the general formula NR³R⁴ with a compound of the general formula (6) or (13):

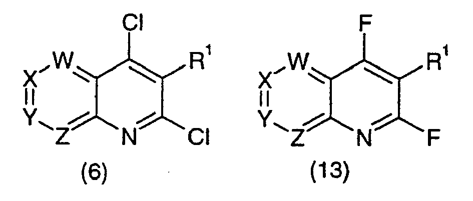

11. The intermediate chemicals having the general formulae (4), (5), (6) and (13): 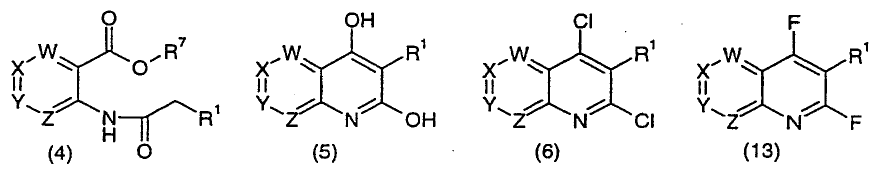 wherein W, X, Y, Z and R¹ are as defined in claim 1 and R⁷ is C₁₋₄ alkyl, other than those compounds of the general formula (5) wherein W and Y are both CH and X and Z are both N and R¹ is methyl, ethyl or phenyl, and other than those compounds of the general formula (5) wherein W is CH, Y is CH₃-C and X and Z are both N and R¹ is methyl, ethyl or phenyl, and other than the compound of the general formula (4) wherein W and Y are both CH₃-C and X and Z are both N and R¹ is methyl and R⁷ is ethyl.

12. A plant fungicidal composition comprising a fungicidally effective amount of a compound as defined in claim 1 and a suitable carrier or diluent therefor.

13. A method of combating or controlling phytopathogenic fungi which comprises applying to a plant, to a seed of a plant, to the locus of the plant or seed or to soil or to any other plant growth medium, a fungicidally effective amount of a compound according to claim 1 or a composition according to claim 12.

## Patentansprüche

1. Verbindung der allgemeinen Formel (1): 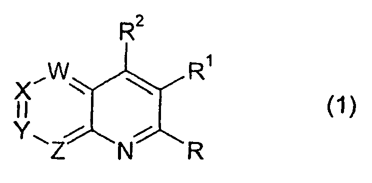 worin
W und Y beide N darstellen und X und Z beide CR⁸ darstellen oder X und Z beide N darstellen und W und Y beide CR⁸ darstellen;
R⁸ H, Halogen, C₁₋₄-Alkyl, C₁₋₄-Alkoxy oder Halogen-(C₁₋₄)-alkyl darstellt;
R und R² unabhängig H, Halogen, C₁₋₈-Alkyl, C₁₋₈-Alkoxy, C₁₋₈-Alkylthio, C₂₋₈-Alkenyl, C₂₋₈-Alkinyl, Cyano öder NR³R⁴ darstellen, mit der Maßgabe, dass mindestens einer von R und R² NR³R⁴ darstellt;
R¹ Halogen, C₁₋₈-Alkyl, C₂₋₈-Alkenyl, C₂₋₈-Alkinyl, C₃₋₈-Cycloalkyl, C₃₋₈-Cycloalkyl-(C₁₋₆)-alkyl, C₁₋₈-Alkoxy, C₁₋₈-Alkylthio, Aryl, Aryloxy, Arylthio, Heteroaryl, Heteroaryloxy, Heteroarylthio, Aryl-(C₁₋₄)-alkyl, Aryl-(C₁₋₄)-alkoxy, Heteroaryl-(C₁₋₄)-alkyl, Heteroaryl-(C₁₋₄)-alkoxy, Aryl-(C₁₋₄)-alkylthio, Heteroaryl-(C₁₋₄)-alkylthio, Morpholino, Piperidino oder Pyrrolidino darstellt;
R³ und R⁴ unabhängig H, C₁₋₈-Alkyl, C₂₋₈-Alkenyl, C₂₋₈-Alkinyl, Aryl, Aryl-(C₁₋₈)-alkyl, C₃₋₈-Cycloalkyl, C₃₋₈-Cycloalkyl-(C₁₋₆)-alkyl, Heteroaryl, Heteroaryl-(C₁₋₈)-alkyl, NR⁵R⁶, darstellen, mit der Maßgabe, dass nicht beide R³ und R⁴ H oder NR⁵R⁶ darstellen, oder
R³ und R⁴ zusammen eine C₃₋₇-Alkylen- oder C₃₋₇-Alkenylenkette, gegebenenfalls substituiert mit einer oder mehreren C₁₋₄-Alkyl- oder C₁₋₄-Alkoxygruppen, bilden, oder, zusammen mit dem Stickstoffatom, an das sie gebunden sind, R³ und R⁴ einen Morpholin-, Thiomorpholin-, Thiomorpholin-S-oxid- oder Thiomorpholin-S-dioxidring oder einen Piperazin- oder Piperazin-N-(C₁₋₄)-alkyl- (insbesondere N-Methyl-)-Ring bilden; und
R⁵ und R⁶ unabhängig H, C₁₋₈-Alkyl, C₂₋₈-Alkenyl, C₂₋₈-Alkinyl, Aryl, Aryl-(C₁₋₈)-alkyl, C₃₋₈-Cycloalkyl, C₃₋₈-Cycloalkyl-(C₁₋₆)-alkyl, Heteroaryl oder Heteroaryl-(C₁₋₈)-alkyl darstellen;
wobei beliebige der vorangehenden Alkyl-, Alkenyl-, Alkinyl- oder Cycloalkylgruppen oder -einheiten (die anders als R⁸ sind), gegebenenfalls substituiert sind mit Halogen, Cyano, C₁₋₆-Alkoxy, C₁₋₆-Alkylcarbonyl, C₁₋₆-Alkoxycarbonyl, C₁₋₆-Halogenalkoxy, C₁₋₆-Alkylthio, Tri-(C₁₋₄)-alkylsilyl, C₁₋₆-Alkylamino oder C₁₋₆-Dialkylamino,
beliebige der vorangehenden Morpholin-, Thiomorpholin-, Piperidin-, Piperazin- und Pyrrolidinringe, gegebenenfalls substituiert sind mit C₁₋₄-Alkyl (insbesondere Methyl), und beliebige der vorangehenden Aryl- oder Heteroarylgruppen oder -einheiten gegebenenfalls substituiert sind mit einem oder mehreren Substituenten, ausgewählt aus Halogen, Hydroxy, Mercapto, C₁₋₆-Alkyl, C₂₋₆-Alkenyl, C₂₋₆-Alkinyl, C₁₋₆-Alkoxy, C₂₋₆-Alkenyloxy, C₂₋₆-Alkinyloxy, Halogen-(C₁₋₆)-alkyl, Halogen-(C₁₋₆)-alkoxy, C₁₋₆-Alkylthio, Halogen-(C₁₋₆)-alkylthio, Hydroxy-(C₁₋₆)-alkyl, C₁₋₄-Alkoxy-(C₁₋₆)-alkyl, C₃₋₆-Cycloalkyl, C₃₋₆-Cycloalkyl-(C₁₋₄)-alkyl, Phenoxy, Benzyloxy, Benzoyloxy, Cyano, Isocyano, Thiocyanato, Isothiocyanato, Nitro, -NR"'R"", -NHCOR"', -NHCONR"'R"", -CONR"'R"", -SO₂R"', -OSO₂R"', -COR"', -CR"'=NR"" oder -N=CR"'R"", worin R"' und R"" unabhängig Wasserstoff, C₁₋₄-Alkyl, Halogen-(C₁₋₄)-alkyl, C₁₋₄-Alkoxy, Halogen-(C₁₋₄)-alkoxy, C₁₋₄-Alkylthio, C₃₋₆-Cycloalkyl, C₃₋₆-Cycloalkyl-(C₁₋₄)-alkyl, Phenyl oder Benzyl darstellen, wobei die Phenyl- und Benzylgruppen gegebenenfalls mit Halogen, C₁₋₄-Alkyl oder C₁₋₄-Alkoxy substituiert sind;
mit der Maßgabe, dass Y nicht CCH₃ darstellt, wenn W CH darstellt, X und Z N darstellen, R NHCH₃ darstellt, R¹ 2,6-Dichlorphenyl darstellt und R² H darstellt.

2. Verbindung nach Anspruch 1, worin W und Y beide N darstellen und X und Z beide CH darstellen oder X und Z beide N darstellen und W und Y beide CH darstellen.

3. Verbindung nach Anspruch 1 oder 2, worin R² NR³R⁴ darstellt.

4. Verbindung nach Anspruch 3, worin R Halogen darstellt.

5. Verbindung nach einem der vorangehenden Ansprüche, worin
R³ C₁₋₈-Alkyl, Halogen-(C₁₋₈)-alkyl, Hydroxy-(C₁₋₈)-alkyl, C₁₋₄-Alkoxy-(C₁₋₈)-alkyl, C₁₋₄-Alkoxyhalogen-(C₁₋₈)-alkyl, Tri-(C₁₋₄)-alkylsilyl-(C₁₋₆)-alkyl, C₁₋₄-Alkylcarbonyl-(C₁₋₈)-alkyl, C₁₋₄-Alkylcarbonylhalogen-(C₁₋₈)-alkyl, Phenyl-(C₁₋₄)-alkyl, C₂₋₈-Alkenyl, Halogen-(C₂₋₈)-alkenyl, C₂₋₈-Alkinyl, C₃₋₈-Cycloalkyl, gegebenenfalls substituiert mit Chlor, Fluor oder Methyl, C₃₋₈-Cycloalkyl-(C₁₋₄)-alkyl, Phenylamino, Piperidino oder Morpholino, wobei der Phenylring von Phenylalkyl oder Phenylamino gegebenenfalls mit einem, zwei oder drei Substituenten, ausgewählt aus Halogen, C₁₋₄-Alkyl, Halogen-(C₁₋₄)-alkyl, C₁₋₄-Alkoxy und Halogen-(C₁₋₄)-alkoxy, substituiert ist, darstellt; und
R⁴ H, C₁₋₄-Alkyl, Halogen-(C₁₋₄)-alkyl oder Amino darstellt, oder
R³ und R⁴ zusammen eine C₃₋₇-Alkylen- oder -Alkenylenkette, gegebenenfalls substituiert mit Methyl, bilden, oder,
zusammen mit dem Stickstoffatom, an das sie gebunden sind, R³ und R⁴ einen Morpholin-, Thiomorpholin-, Thiomorpholin-S-oxid- oder Thiomorpholin-S-dioxidring oder einen Piperazin- oder Piperazin-N-(C₁₋₄)-alkyl- (insbesondere N-Methyl-)-Ring bilden, worin die Morpholin- oder Piperazinringe gegebenenfalls mit Methyl substituiert sind.

6. Verbindung nach einem der vorangehenden Ansprüche, worin
R¹ Phenyl, gegebenenfalls substituiert mit einem bis fünf Halogenatomen, oder mit einem bis drei Substituenten, ausgewählt aus Halogen, C₁₋₄-Alkyl, Halogen-(C₁₋₄)-alkyl, C₁₋₄-Alkoxy oder Halogen-(C₁₋₄)-alkoxy, Pyridyl, gegebenenfalls substituiert mit einem bis vier Halogenatomen oder mit einem bis drei Substituenten, ausgewählt aus Halogen, C₁₋₄-Alkyl, Halogen-(C₁₋₄)-alkyl, C₁₋₄-Alkoxy oder Halogen-(C₁₋₄)-alkoxy, 2- oder 3-Thienyl, gegebenenfalls substituiert mit einem bis drei Halogenatomen, oder mit einem bis drei Substituenten, ausgewählt aus Halogen, C₁₋₄-Alkyl, Halogen-(C₁₋₄)-alkyl, C₁₋₄-Alkoxy oder Halogen-(C₁₋₄)-alkoxy, oder Piperidino oder Morpholino, beide gegebenenfalls substituiert mit einer oder zwei Methylgruppen, darstellt.

7. Verbindung nach Anspruch 6, worin R¹ 2,6-Difluorphenyl, 2-Fluor-6-chlorphenyl, 2,5,6-Trifluorphenyl, 2,4,6-Trifluorphenyl, 2,6-Difluor-4-methoxyphenyl oder Pentafluorphenyl darstellt.

8. Verbindung nach Anspruch 1, worin
W und Y beide N darstellen und X und Z beide CR⁸ darstellen oder X und Z beide N darstellen und W und Y beide CR⁸ darstellen;
R⁸ H, Halogen, C₁₋₄-Alkyl, C₁₋₄-Alkoxy oder Halogen-(C₁₋₄)-alkyl darstellt;
einer von R und R² (vorzugsweise R²) NR³R⁴ darstellt und der andere Halogen darstellt;
R¹ Halogen, C₁₋₈-Alkyl, C₂₋₈-Alkenyl, C₂₋₈-Alkinyl, C₃₋₈-Cycloalkyl, C₃₋₈-Cycloalkyl-(C₁₋₆)-alkyl, C₁₋₈-Alkoxy, C₁₋₈-Alkylthio, Aryl, Aryloxy, Arylthio, Heteroaryl, Heteroaryloxy, Heteroarylthio, Aryl-(C₁₋₄)-alkyl, Aryl-(C₁₋₄)-alkoxy, Heteroaryl-(C₁₋₄)-alkyl, Heteroaryl-(C₁₋₄)-alkoxy, Aryl-(C₁₋₄)-alkylthio, Heteroaryl-(C₁₋₄)-alkylthio, Morpholino, Piperidino oder Pyrrolidino darstellt;
R³ und R⁴ unabhängig H, C₁₋₈-Alkyl, C₂₋₈-Alkenyl, C₂₋₈-Alkinyl, Aryl, Aryl-(C₁₋₈)-alkyl, C₃₋₈-Cycloalkyl, C₃₋₈-Cycloalkyl-(C₁₋₆)-alkyl, Heteroaryl, Heteroaryl-(C₁₋₈)-alkyl, NR⁵R⁶ darstellen, mit der Maßgabe, dass nicht beide R³ und R⁴ H oder NR⁵R⁶ darstellen, oder
R³ und R⁴ zusammen eine C₃₋₇-Alkylen- oder C₃₋₇-Alkylenkette, gegebenenfalls substituiert mit einer oder mehreren C₁₋₄-Alkyl- oder C₁₋₄-Alkoxygruppen, bilden, oder, zusammen mit dem Stickstoffatom, an das sie gebunden sind, R³ und R⁴ einen Morpholin-, Thiomorpholin-, Thiomorpholin-S-oxid- oder Thiomorpholin-S-dioxidring, oder einen Piperazin- oder Piperazin-N-(C₁₋₄)-alkyl- (insbesondere N-Methyl-)-Ring bilden; und
R⁵ und R⁶ unabhängig H, C₁₋₈-Alkyl, C₂₋₈-Alkenyl, C₂₋₈-Alkinyl, Aryl, Aryl-(C₁₋₈)-alkyl, C₃₋₈-Cycloalkyl, C₃₋₈-Cycloalkyl-(C₁₋₆)-alkyl, Heteroaryl oder Heteroaryl-(C₁₋₈)-alkyl darstellen; wobei beliebige der vorangehenden Alkyl-, Alkenyl-, Alkinyl- oder Cycloalkylgruppen oder -einheiten (die anders als R⁸ sind), gegebenenfalls substituiert sind mit Halogen, Cyano, C₁₋₆-Alkoxy, C₁₋₆-Alkylcarbonyl, C₁₋₆-Alkoxycarbonyl, C₁₋₆-Halogenalkoxy, C₁₋₆-Alkylthio, Tri-(C₁₋₄)-alkylsilyl, C₁₋₆-Alkylamino oder C₁₋₆-Dialkylamino,
beliebige der vorangehenden Morpholin-, Thiomorpholin-, Piperidin-, Piperazin- und Pyrrolidinringe, gegebenenfalls substituiert sind mit C₁₋₄-Alkyl (insbesondere Methyl), und beliebige der Aryl-, Heteroaryl-, Aryloxy- oder Heteroarylgruppen gegebenenfalls substituiert sind mit einem oder mehreren Substituenten, ausgewählt aus Halogen, Hydroxy, Mercapto, C₁₋₆-Alkyl, C₂₋₆-Alkenyl, C₂₋₆-Alkinyl, C₁₋₆-Alkoxy, C₂₋₆-Alkenyloxy, C₂₋₆-Alkinyloxy, Halogen-(C₁₋₆)-alkyl, Halogen-(C₁₋₆)-alkoxy, C₁₋₆-Alkylthio, Halogen-(C₁₋₆)-alkylthio, Hydroxy-(C₁₋₆)-alkyl, C₁₋₄-Alkoxy-(C₁₋₆)-alkyl, C₃₋₆-Cycloalkyl, C₃₋₆-Cycloalkyl-(C₁₋₄)-alkyl, Phenoxy, Benzyloxy, Benzoyloxy, Cyano, Isocyano, Thiocyanato, Isothiocyanato, Nitro, -NR"'R"", -NHCOR"', -NHCONR"'R"", -CONR"'R"", -SO₂R"', -OSO₂R"', -COR"', -CR"'=NR"" oder -N=CR"'R"", worin R"' und R"" unabhängig Wasserstoff, C₁₋₄-Alkyl, Halogen-(C₁₋₄)-alkyl, C₁₋₄-Alkoxy, Halogen-(C₁₋₄)-alkoxy, C₁₋₄-Alkylthio, C₃₋₆-Cycloalkyl, C₃₋₆-Cycloalkyl-(C₁₋₄)-alkyl, Phenyl oder Benzyl darstellen, wobei die Phenyl- und Benzylgruppen gegebenenfalls mit Halogen, C₁₋₄-Alkyl oder C₁₋₄-Alkoxy substituiert sind.

9. Verbindung nach Anspruch 1, worin
W und Y beide N darstellen und X und Z beide CR⁸ darstellen oder X und Z beide N darstellen und W und Y beide CR⁸ darstellen;
R⁸ H, Halogen, C₁₋₄-Alkyl, C₁₋₄-Alkoxy oder Halogen-(C₁₋₄)-alkyl darstellt;
einer von R und R² (vorzugsweise R²) NR³R⁴ darstellt und der andere Halogen darstellt;
R¹ Halogen, C₁₋₈-Alkyl, C₂₋₈-Alkenyl, C₂₋₈-Alkinyl, C₃₋₈-Cycloalkyl, C₃₋₈-Cycloalkyl-(C₁₋₆)-alkyl, C₁₋₈-Alkoxy, C₁₋₈-Alkylthio, Aryl, Aryloxy, Arylthio, Heteroaryl, Heteroaryloxy, Heteroarylthio, Aryl-(C₁₋₄)-alkyl, Aryl-(C₁₋₄)-alkoxy, Heteroaryl-(C₁₋₄)-alkyl, Heteroaryl-(C₁₋₄)-alkoxy, Aryl-(C₁₋₄)-alkylthio, Heteroaryl-(C₁₋₄)-alkylthio, Morpholino, Piperidino oder Pyrrolidino darstellt;
R³ C₁₋₄-Alkyl, Halogen-(C₁₋₄)-alkyl, C₂₋₄-Alkenyl, C₃₋₆-Cycloalkyl, C₃₋₆-Cycloalkyl-(C₁₋₄)-alkyl oder Phenylamino darstellt, worin der Phenylring gegebenenfalls mit einem, zwei oder drei Substituenten, ausgewählt aus Halogen, C₁₋₄-Alkyl, Halogen-(C₁₋₄)-alkyl, C₁₋₄-Alkoxy und Halogen-(C₁₋₄)-alkoxy, substituiert ist; und R⁴ H, C₁₋₄-Alkyl oder Amino darstellt, oder
R³ und R⁴ zusammen eine C₄₋₆-Alkylenkette, gegebenenfalls substituiert mit C₁₋₄-Alkyl oder C₁₋₄-Alkoxy, bilden, oder, zusammen mit dem Stickstoffatom, an das sie gebunden sind, R³ und R⁴ einen Morpholin-, Thiomorpholin-, Thiomorpholin-S-oxid- oder Thiomorpholin-S-dioxidring, oder einen Piperazin- oder Piperazin-N-(C₁₋₄)-alkyl- (insbesondere N-Methyl-)-Ring bilden;
wobei beliebige der Alkyl-, Alkenyl-, Alkinyl- oder Cycloalkylgruppen oder -einheiten (die anders als R⁸ sind), gegebenenfalls substituiert sind mit Halogen, Cyano, C₁₋₆-Alkoxy, C₁₋₆-Alkylcarbonyl, C₁₋₆-Alkoxycarbonyl, C₁₋₆-Halogenalkoxy, C₁₋₆-Alkylthio, Tri-(C₁₋₄)-alkylsilyl, C₁₋₆-Alkylamino oder C₁₋₆-Dialkylamino,
beliebige der vorangehenden Morpholin-, Thiomorpholin-, Piperidin-, Piperazin- und Pyrrolidinringe, gegebenenfalls substituiert sind mit C₁₋₄-Alkyl (insbesondere Methyl), und beliebige der Aryl- oder Heteroarylgruppen oder -einheiten gegebenenfalls substituiert sind mit einem oder mehreren Substituenten, ausgewählt aus Halogen, Hydroxy, Mercapto, C₁₋₆-Alkyl, C₂₋₆-Alkenyl, C₂₋₆-Alkinyl, C₁₋₆-Alkoxy, C₂₋₆-Alkenyloxy, C₂₋₆-Alkinyloxy, Halogen-(C₁₋₆)-alkyl, Halogen-(C₁₋₆)-alkoxy, C₁₋₆-Alkylthio, Halogen-(C₁₋₆)-alkylthio, Hydroxy-(C₁₋₆)-alkyl, C₁₋₄-Alkoxy-(C₁₋₆)-alkyl, C₃₋₆-Cycloalkyl, C₃₋₆-Cycloalkyl-(C₁₋₄)-alkyl, Phenoxy, Benzyloxy, Benzoyloxy, Cyano, Isocyano, Thiocyanato, Isothiocyanato, Nitro, -NR"'R"", -NHCOR"', -NHCONR"'R"", -CONR"'R"", -SO₂R"', -OSO₂R"', -COR"', -CR"'=NR"" oder -N=CR"'R"", worin R"' und R"" unabhängig Wasserstoff, C₁₋₄-Alkyl, Halogen-(C₁₋₄)-alkyl, C₁₋₄-Alkoxy, Halogen-(C₁₋₄)-alkoxy, C₁₋₄-Alkylthio, C₃₋₆-Cycloalkyl, C₃₋₆-Cycloalkyl-(C₁₋₄)-alkyl, Phenyl oder Benzyl darstellen, wobei die Phenyl- und Benzylgruppen gegebenenfalls mit Halogen, C₁₋₄-Alkyl oder C₁₋₄-Alkoxy substituiert sind.

10. Verfahren zur Herstellung einer Verbindung der allgemeinen Formel (1) nach Anspruch 1, wobei einer von R und R² Chlor oder Fluor darstellt und der andere NR³R⁴ darstellt, und W, X, Y, Z, R¹, R³ und R⁴ wie in Anspruch 1 definiert sind, das Umsetzen eines Amins der allgemeinen Formel NR³R⁴ mit einer Verbindung der allgemeinen Formel (6) oder (13) : 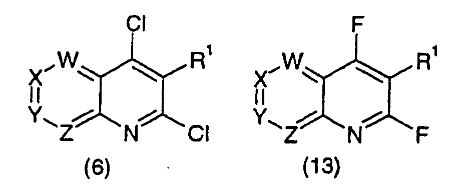 umfasst.

11. Zwischenproduktchemikalien mit den allgemeinen Formeln (4), (5), (6) und (13): 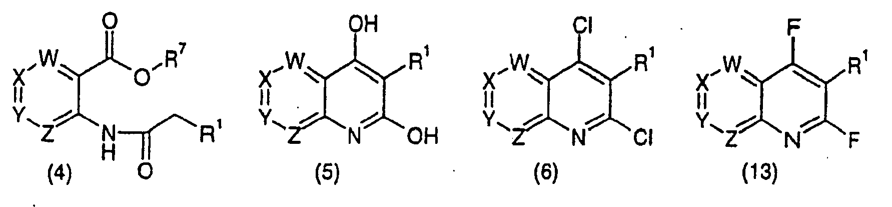 worin W, X, Y, Z und R¹ wie in Anspruch 1 definiert sind, und R⁷ C₁₋₄-Alkyl darstellt, außer jenen Verbindungen der allgemeinen Formel (5), worin W und Y beide CH darstellen und X und Z beide N darstellen, und R¹ Methyl, Ethyl oder Phenyl darstellt, und außer jenen Verbindungen der allgemeinen Formel (5), worin W CH darstellt, Y CH₃-C darstellt und X und Z beide N darstellen und R¹ Methyl, Ethyl oder Phenyl darstellt, und außer der Verbindung der allgemeinen Formel (4), worin W und Y beide CH₃-C darstellen und X und Z beide N darstellen und R¹ Methyl darstellt und R⁷ Ethyl darstellt.

12. Pflanzen-fungizide Zusammensetzung, umfassend eine fungizid wirksame Menge einer Verbindung, wie in Anspruch 1 definiert, und einen geeigneten Träger oder ein geeignetes Verdünnungsmittel dafür.

13. Verfahren zum Bekämpfen oder Eindämmen von phytopathogenen Pilzen, das Applizieren auf eine Pflanze, auf ein Saatgut der Pflanze, auf den Standort der Pflanze oder des Saatguts, oder auf den Boden oder beliebiges anderes Pflanzenwachstumsmedium einer fungizid wirksamen Menge einer Verbindung nach Anspruch 1 oder einer Zusammensetzung nach Anspruch 12 umfasst.

## Revendications

1. Composé de formule générale (1) 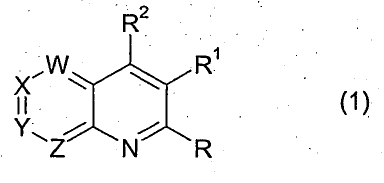 dans laquelle
W et Y représentent tous les deux N et X et Z représentent tous les deux CR⁸ ou X et Z représentent tous les deux N et W et Y représentent tous les deux CR⁸ ;
R⁸ représente H, un groupe halogéno, alkyle en C₁ à C₄, alcoxy en C₁ à C₄ ou halogénoalkyle en C₁ à C₄ ;
R et R² représentent indépendamment H, un groupe halogéno, alkyle en C₁ à C₈, alcoxy en C₁ à C₈, alkyl (en C₁ à C₈)thio, alcényle en C₂ à C₈, alcynyle en C₂ à C₈, cyano ou NR³R⁴, à condition qu'au moins l'un de R et R² représente NR³R⁴ ;
R¹ représente un groupe halogéno, alkyle en C₁ à C₈, alcényle en C₂ à C₈, alcynyle en C₂ à C₈, cycloalkyle en C₃ à C₈, cycloalkyl (en C₁ à C₈)alkyle en C₁ à C₆, alcoxy en C₁ à C₈, alkyl(en C₁ à C₈)thio, aryle, aryloxy, arylthio, hétéroaryle, hétéroaryloxy, hétéroarylthio, arylalkyle en C₁ à C₄, arylalcoxy en C₁ à C₄, hétéroarylalkyle en C₁ à C₄, hétéroarylalcoxy en C₁ à C₄, arylalkyl(en C₁ à C₄)thio, hétéroarylalkyl(en C₁ à C₄)thio, morpholino, pipéridino ou pyrrolidino ;
R³ et R⁴ représentent indépendamment H, un groupe alkyle en C₁ à C₈, alcényle en C₂ à C₈, alcynyle en C₂ à C₈, aryle, arylalkyle en C₁ à C₈, cycloalkyle en C₃ à C₈, cycloalkyl(en C₃ à C₈)alkyle en C₁ à C₆, hétéroaryle, hétéroarylalkyle en C₁ à C₈, NR⁵R⁶, à condition que R³ et R⁴ ne représentent pas tous les deux H ou NR⁵R⁶, ou
R³ et R⁴ forment ensemble une chaîne alkylène en C₃ à C₇ ou alcénylène en C₃ à C₇ éventuellement substituée par un ou plusieurs groupes alkyle en C₁ à C₄ ou alcoxy en C₁ à C₄, ou,
conjointement avec l'atome d'azote auquel ils sont fixés, R³ et R⁴ forment un cycle morpholine, thiomorpholine, thiomorpholine S-oxyde ou thiomorpholine S-dioxyde ou un cycle pipérazine ou pipérazine N-alkyle en C₁ à C₄ (spécialement N-méthyle) ; et
R⁵ et R⁶ représentent indépendamment H, un groupe alkyle en C₁ à C₈, alcényle en C₂ à C₈, alcynyle en C₂ à C₈, aryle, arylalkyle en C₁ à C₈, cycloalkyle en C₃ à C₈, cycloalkyl(en C₃ à C₈)alkyle en C₁ à C₆, hétéroaryle ou hétéroarylalkyle en C₁ à C₈ ;
l'un quelconque des groupes ou radicaux précédents alkyle, alcényle, alcynyle ou cycloalkyle (autre que pour R⁸) étant éventuellement substitué par un atome d'halogène, un groupe cyano, alcoxy en C₁ à C₆, alkyl (en C₁ à C₆) - carbonyle, alcoxy(en C₁ à C₆)carbonyle, halogénoalcoxy en C₁ à C₆, alkyl(en C₁ à C₆)thio, trialkyl(en C₁ à C₄)silyle, alkyl(en C₁ à C₆)amino ou dialkyl(en C₁ à C₆)amino,
l'un quelconque des cycles précédents morpholine, thiomorpholine, pipéridine, pipérazine et pyrrolidine étant éventuellement substitué par un groupe alkyle en C₁ à C₄ (spécialement méthyle), et
l'un quelconque des groupes ou radicaux précédents aryle ou hétéroaryle étant éventuellement substitué par un ou plusieurs substituants choisis parmi les groupes halogéno, hydroxy, mercapto, alkyle en C₁ à C₆, alcényle en C₂ à C₆, alcynyle en C₂ à C₆, alcoxy en C₁ à C₆, alcényl-(en C₂ à C₆)oxy, alcynyl(en C₂ à C₆)oxy, halogénoalkyle en C₁ à C₆, halogénoalcoxy en C₁ à C₆, alkyl(en C₁ à C₆)thio, halogénoalkyl(en C₁ à C₆)thio, hydroxyalkyle en C₁ à C₆, alcoxy(en C₁ à C₄)alkyle en C₁ à C₆, cycloalkyle en C₃ à C₆, cycloalkyl(en C₃ à C₆)alkyle en C₁ à C₄, phénoxy, benzyloxy, benzoyloxy, cyano, isocyano, thiocyanato, isothiocyanato, nitro,-NR"'R"", -NHCOR"', -NHCONR"'R"", -CONR"'R"", -SO₂R"', -OSO₂R"', -COR"', -CR"'=NR"" ou -N=CR"'R"", où R"' et R"" représentent indépendamment un atome d'hydro-gène, un groupe alkyle en C₁ à C₄, halogéno-alkyle en C₁ à C₄, alcoxy en C₁ à C₄, halogéno-alcoxy en C₁ à C₄, alkyl(en C₁ à C₄)thio, cyclo-alkyle en C₃ à C₆, cycloalkyl(en C₃ à C₆)alkyle en C₁ à C₄, phényle ou benzyle, les groupes phényle et benzyle étant éventuellement substitués par un atome d'halogène, un groupe alkyle en C₁ à C₄ ou alcoxy en C₁ à C₄ ;
à condition que Y ne représente pas CCH₃ lorsque W représente CH, X et Z représentent N, R représente NHCH₃, R¹ représente un groupe 2,6-dichlorophényle et R² représente H.

2. Composé selon la revendication 1, dans lequel W et Y représentent tous les deux N et X et Z représentent tous les deux CH ou X et Z représentent tous les deux N et W et Y représentent tous les deux CH.

3. Composé selon la revendication 1 ou 2, dans lequel R² représente NR³R⁴.

4. Composé selon la revendication 3, dans lequel R représente un groupe halogéno.

5. Composé selon l'une quelconque des revendications précédentes dans lequel
R³ représente un groupe alkyle en C₁ à C₈, halogénoalkyle en C₁ à C₈, hydroxyalkyle en C₁ à C₈, alcoxy(en C₁ à C₄)alkyle en C₁ à C₈, alcoxy(en C₁ à C₄)-halogénoalkyle en C₁ à C₈, trialkyl(en C₁ à C₄)silyl-alkyle en C₁ à C₆, alkyl(en C₁ à C₄)carbonylalkyle en C₁ à C₈, alkyl(en C₁ à C₄)carbonylhalogénoalkyle en C₁ à C₈, phénylalkyle en C₁ à C₄, alcényle en C₂ à C₈, halogénoalcényle en C₂ à C₈, alcynyle en C₂ à C₈, cycloalkyle en C₃ à C₈ éventuellement substitué par un groupe chloro, fluoro ou méthyle, cycloalkyl(en C₃ à C₈)alkyle en C₁ à C₄, phénylamino, pipéridino ou morpholino, le cycle phényle des groupes phénylalkyle ou phénylamino étant éventuellement substitué par un, deux ou trois substituants choisis parmi un groupe halogéno, alkyle en C₁ à C₄, halogénoalkyle en C₁ à C₄, alcoxy en C₁ à C₄ et halogénoalcoxy en C₁ à C₄ ; et
R⁴ représente H, un groupe alkyle en C₁ à C₄ ou amino, ou
R³ et R⁴ forment ensemble une chaîne alkylène ou alcénylène en C₃ à C₇ éventuellement substituée par un groupe méthyle, ou,
conjointement avec l'atome d'azote auquel ils sont fixés, R³ et R⁴ forment un cycle morpholine, thiomorpholine, thiomorpholine S-oxyde ou thiomorpholine S-dioxyde ou un cycle pipérazine ou pipérazine N-alkyle en C₁ à C₄ (spécialement N-méthyle), où les cycles morpholine ou pip-razine sont éventuellement substitués par un groupe méthyle.

6. Composé selon l'une quelconque des revendications précédentes dans lequel
R¹ représente un groupe phényle éventuellement substitué par un à cinq atomes d'halogène ou un à trois substituants choisis parmi un groupe halogéno, alkyle en C₁ à C₄, halogénoalkyle en C₁ à C₄, alcoxy en C₁ à C₄ ou halogénoalcoxy en C₁ à C₄, pyridyle éventuellement substitué par un à quatre atomes d'halogène ou par un à trois substituants choisis parmi un groupe halogéno, alkyle en C₁ à C₄, halogénoalkyle en C₁ à C₄, alcoxy en C₁ à C₄ ou halogénoalcoxy en C₁ à C₄, 2- ou 3-thiényle éventuellement substitué par un à trois atomes d'halogène ou par un à trois substituants choisis parmi un groupe halogéno, alkyle en C₁ à C₄, halogénoalkyle en C₁ à C₄, alcoxy en C₁ à C₄ ou halogénoalcoxy en C₁ à C₄, ou pipéridino ou morpholino tous les deux éventuellement substitués par un ou deux groupes méthyle.

7. Composé selon la revendication 6 dans lequel R¹ représente un groupe 2,6-difluorophényle, 2-fluoro-6-chlorophényle, 2,5,6-trifluorophényle, 2,4,6-trifluoro-phényle, 2,6-difluoro-4-méthoxy-phényle ou pentafluoro-phényle.

8. Composé selon la revendication 1 dans lequel
W et Y représentent tous les deux N et X et Z représentent tous les deux CR⁸ ou X et Z représentent tous les deux N et W et Y représentent tous les deux CR⁸ ;
R⁸ représente H, un groupe halogéno, alkyle en C₁ à C₄, alcoxy en C₁ à C₄ ou halogénoalkyle en C₁ à C₄ ;
l'un de R et R² (de préférence R²) représente NR³R⁴ et l'autre représente un groupe halogéno ;
R¹ représente un groupe halogéno, alkyle en C₁ à C₈, alcényle en C₂ à C₈, alcynyle en C₂ à C₈, cycloalkyle en C₃ à C₈, cycloalkyl(en C₃ à C₈)alkyle en C₁ à C₆, alcoxy en C₁ à C₈, alkyl(en C₁ à C₈)thio, aryle, aryloxy, arylthio, hétéroaryle, hétéroaryloxy, hétéroarylthio, arylalkyle en C₁ à C₄, arylalcoxy en C₁ à C₄, hétéroarylalkyle en C₁ à C₄, hétéroarylalcoxy en C₁ à C₄, arylalkyl(en C₁ à C₄)thio, hétéroarylalkyl(en C₁ à C₄)thio, morpholino, pipéridino ou pyrrolidino ;
R³ et R⁴ représentent indépendamment H, un groupe alkyle en C₁ à C₈, alcényle en C₂ à C₈, alcynyle en C₂ à C₈, aryle, arylalkyle en C₁ à C₈, cycloalkyle en C₃ à C₈, cycloalkyl(en C₃ à C₈)alkyle en C₁ à C₆, hétéroaryle, hétéroarylalkyle en C₁ à C₈, NR⁵R⁶, à condition que R³ et R⁴ ne représentent pas tous les deux H ou NR⁵R⁶, ou R³ et R⁴ forment ensemble une chaîne alkylène en C₃ à C₇ ou alcénylène en C₃ à C₇ éventuellement substituée par un ou plusieurs groupes alkyle en C₁ à C₄ ou alcoxy en C₁ à C₄, ou,
conjointement avec l'atome d'azote auquel ils sont fixés, R³ et R⁴ forment un cycle morpholine, thiomorpholine, thiomorpholine S-oxyde ou thiomorpholine S-dioxyde ou un cycle pipérazine ou pipérazine N-alkyle en C₁ à C₄ (spécialement N-méthyle) ; et
R⁵ et R⁶ représentent indépendamment H, un groupe alkyle en C₁ à C₈, alcényle en C₂ à C₈, alcynyle en C₂ à C₈, aryle, arylalkyle en C₁ à C₈, cycloalkyle en C₃ à C₈, cycloalkyl(en C₃ à C₈)alkyle en C₁ à C₆, hétéroaryle ou hétéroarylalkyle en C₁ à C₈ ;
l'un quelconque des groupes ou radicaux précédents alkyle, alcényle, alcynyle ou cycloalkyle (autre que pour R⁸) étant éventuellement substitué par un atome d'halogène, un groupe cyano, alcoxy en C₁ à C₆, alkyl(en C₁ à C₆)carbonyle, alcoxy(en C₁ à C₆)carbonyle, halogénoalcoxy en C₁ à C₆, alkyl(en C₁ à C₆)thio, trialkyl(en C₁ à C₄)silyle, alkyl(en C₁ à C₆)amino ou dialkyl(en C₁ à C₆)amino,
l'un quelconque des cycles précédents morpholine, thiomorpholine, pipéridine, pipérazine et pyrrolidine étant éventuellement substitué par un groupe alkyle en C₁ à C₄ (spécialement méthyle), et
l'un quelconque des groupes aryle, hétéroaryle, aryloxy ou hétéroaryle précédents étant éventuellement substitué par un ou plusieurs substituants choisis parmi les groupes halogéno, hydroxy, mercapto, alkyle en C₁ à C₆, alcényle en C₂ à C₆, alcynyle en C₂ à C₆, alcoxy en C₁ à C₆, alcényl(en C₂ à C₆)oxy, alcynyl(en C₂ à C₆)oxy, halogénoalkyle en C₁ à C₆, halogénoalcoxy en C₁ à C₆, alkyl (en C₁ à C₆)thio, halogénoalkyl-(en C₁ à C₆)thio, hydroxyalkyle en C₁ à C₆, alcoxy(en C₁ à C₄)alkyle en C₁ à C₆, cycloalkyle en C₃ à C₆, cycloalkyl (en C₃ à C₆)alkyle en C₁ à C₄, phénoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" ou -N=CR"'R"", où R"' et R"" représentent indépendamment un atome d'hydrogène, un groupe alkyle en C₁ à C₄, halogénoalkyle en C₁ à C₄, alcoxy en C₁ à C₄, halogénoalcoxy en C₁ à C₄, alkyl(en C₁ à C₄)thio, cycloalkyle en C₃ à C₆, cycloalkyl(en C₃ à C₆)alkyle en C₁ à C₄, phényle ou benzyle, les groupes phényle et benzyle étant éventuellement substitués par un atome d'halogène, un groupe alkyle en C₁ à C₄ ou alcoxy en C₁ à C₄.

9. Composé selon la revendication 1 dans lequel
W et Y représentent tous les deux N et X et Z représentent tous les deux CR⁸ ou X et Z représentent tous les deux N et W et Y représentent tous les deux CR⁸ ;
R⁸ représente H, un groupe halogéno, alkyle en C₁ à C₄, alcoxy en C₁ à C₄ ou halogénoalkyle en C₁ à C₄ ;
l'un de R et R² (de préférence R²) représente NR³R⁴ et l'autre représente un groupe halogéno ;
R¹ représente un groupe halogéno, alkyle en C₁ à C₈, alcényle en C₂ à C₈, alcynyle en C₂ à C₈, cycloalkyle en C₃ à C₈, cycloalkyl(en C₃ à C₈)alkyle en C₁ à C₆, alcoxy en C₁ à C₈, alkyl(en C₁ à C₈)thio, aryle, aryloxy, arylthio, hétéroaryle, hétéroaryloxy, hétéroarylthio, arylalkyle en C₁ à C₄, arylalcoxy en C₁ à C₄, hétéroarylalkyle en C₁ à C₄, hétéroarylalcoxy en C₁ à C₄, arylalkyl(en C₁ à C₄)thio, hétéroarylalkyl (en C₁ à C₄)thio, morpholino, pipéridino ou pyrrolidino ;
R³ représente un groupe alkyle en C₁ à C₄, halogénoalkyle en C₁ à C₄, alcényle en C₂ à C₄, cycloalkyle en C₃ à C₆, cycloalkyl (en C₃ à C₆) - alkyle en C₁ à C₄ ou phénylamino dans lequel le cycle phényle est éventuellement substitué par un, deux ou trois substituants choisis parmi un groupe halogéno, alkyle en C₁ à C₄, halogénoalkyle en C₁ à C₄, alcoxy en C₁ à C₄, et halogénoalcoxy en C₁ à C₄ ; et R⁴ représente H, un groupe alkyle en C₁ à C₄ ou amino, ou
R³ et R⁴ forment ensemble une chaîne alkylène en C₄ à C₆ éventuellement substituée par un groupe alkyle en C₁ à C₄ ou alcoxy en C₁ à C₄, ou,
conjointement avec l'atome d'azote auquel ils sont fixés, R³ et R⁴ forment un cycle morpholine, thiomorpholine, thiomorpholine *S*-oxyde ou thiomorpholine *S*-dioxyde ou un cycle pipérazine ou pipérazine *N*-alkyle en C₁ à C₄ (spécialement *N*-méthyle) ;
l'un quelconque des groupes ou radicaux précédents alkyle, alcényle, alcynyle ou cycloalkyle (autre que pour R⁸) étant éventuellement substitué par un atome d'halogène, un groupe cyano, alcoxy en C₁ à C₆, alkyl (en C₁ à C₆)carbonyle, alcoxy(en C₁ à C₆)carbonyle, halogénoalcoxy en C₁ à C₆, alkyl(en C₁ à C₆)thio, trialkyl(en C₁ à C₄)silyle, alkyl(en C₁ à C₆)amino ou dialkyl(en C₁ à C₆)amino,
l'un quelconque des cycles précédents morpholine, thiomorpholine, pipéridine, pipérazine et pyrrolidine étant éventuellement substitué par un groupe alkyle en C₁ à C₄ (spécialement méthyle), et
l'un quelconque des groupes ou radicaux précédents aryle ou hétéroaryle étant éventuellement substitué par un ou plusieurs substituants choisis parmi les groupes halogéno, hydroxy, mercapto, alkyle en C₁ à C₆, alcényle en C₂ à C₆, alcynyle en C₂ à C₆, alcoxy en C₁ à C₆, alcényl (en C₂ à C₆)oxy, alcynyl(en C₂ à C₆)oxy, halogénoalkyle en C₁ à C₆, halogénoalcoxy en C₁ à C₆, alkyl(en C₁ à C₆)thio, halogénoalkyl (en C₁ à C₆)thio, hydroxyalkyle en C₁ à C₆, alcoxy(en C₁ à C₄)alkyle en C₁ à C₆, cycloalkyle en C₃ à C₆, cycloalkyl(en C₃ à C₆)alkyle en C₁ à C₄, phénoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" ou -N=CR"'R"", où R"' et R"" représentent indépendamment un atome d'hydrogène, un groupe alkyle en C₁ à C₄, halogénoalkyle en C₁ à C₄, alcoxy en C₁ à C₄, halogénoalcoxy en C₁ à C₄, alkyl(en C₁ à C₄)thio, cycloalkyle en C₃ à C₆, cycloalkyl(en C₃ à C₆) - alkyle en C₁ à C₄, phényle ou benzyle, les groupes phényle et benzyle étant éventuellement substitués par un atome d'halogène, un groupe alkyle en C₁ à C₄ ou alcoxy en C₁ à C₄.

10. Procédé de préparation d'un composé de formule générale (1) selon la revendication 1 dans lequel l'un de R et R² représente un groupe chloro ou un fluoro et l'autre représente NR³R⁴ et W, X, Y, Z, R¹, R³ et R⁴ sont tels que définis dans la revendication 1, qui comprend la réaction d'une amine de formule générale NR³R⁴ avec un composé de formule générale (6) ou (13) :

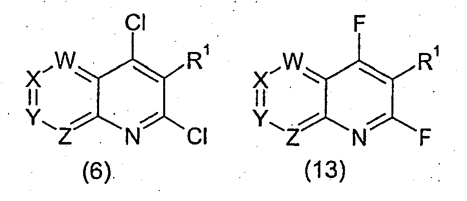

11. Produits chimiques intermédiaires ayant les formules générales (4), (5), (6) et (13) : 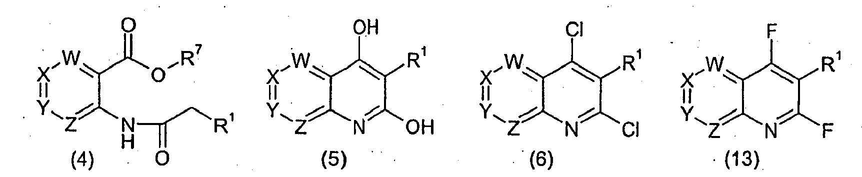 dans lesquelles W, X, Y, Z et R¹ sont tels que définis dans la revendication 1 et R⁷ représente un groupe alkyle en C₁ à C₄, autres que ces composés de formule générale (5) dans laquelle W et Y représentent tous les deux CH et X et Z représentent tous les deux N et R¹ représente un groupe méthyle, éthyle ou phényle, et autres que ces composés de formule générale (5) dans laquelle W représente CH, Y représente CH₃-C et X et Z représentent tous les deux N et R¹ représente un groupe méthyle, éthyle ou phényle, et autre que le composé de formule générale (4) dans laquelle W et Y représentent tous les deux CH₃-C et X et Z représentent tous les deux N et R¹ représente un groupe méthyle et R⁷ représente un groupe éthyle.

12. Composition fongicide pour plantes comprenant une quantité efficace d'un point de vue fongicide d'un composé tel que défini dans la revendication 1 et un support ou un diluant approprié de celui-ci.

13. Procédé de lutte contre ou de contrôle des champignons phytopathogènes, qui comprend l'application sur une plante, sur une graine d'une plante, sur le lieu de la plante ou de la graine ou sur la terre ou sur tout autre milieu de croissance de la plante, d'une quantité efficace d'un point de vue fongicide d'un composé selon la revendication 1 ou d'une composition selon la revendication 12.
